# EUROPÄISCHE PATENTANMELDUNG

(11) **EP 3 591 401 A1**
(43) Veröffentlichungstag der Anmeldung: **08.01.2020**
(21) Anmeldenummer: 18182266.9
(22) Anmeldetag: 06.07.2018
(51) Int. Cl.: G01N 33/543, G01N 33/58, G01N 33/68

(54) **VERFAHREN ZUM AUTOMATISIERTEN DETEKTIEREN VON ANTIKÖRPERN IN EINER FLÜSSIGEN BIOLOGISCHEN PROBE UNTER VERWENDUNG EINES ANTIGEN-CHIPS UND ANTIGEN-CHIP HIERFÜR**

(71) Anmelder: Euroimmun Medizinische Labordiagnostika AG, 23560 Lübeck (DE)
(72) Erfinder: Morgenroth, Katja, 23923 Herrnburg (DE); Viertel, Vanessa, 23554 Lübeck (DE); Steller, Ulf, 21244 Buchholz (DE); Gerlach, Stefan, 23627 Groß Grönau (DE); Marzahl, Christian, 23552 Lübeck (DE); Voigt, Jörn, 23558 Lübeck (DE); Stöcker, Winfried, 23627 Groß Grönau (DE)

(57) **Zusammenfassung**

Vorgeschlagen wird ein Verfahren zum automatisierten Detektieren von Antikörpern in einer flüssigen biologischen Probe mittels Verwendung eines Antigen-Chips mit darauf aufgebrachten Antigen-Spots, welche einen gleichen, gemeinsamen Farbstoff aufweisen. Die Antigen-Spots bilden jeweilige Antigen-Spot-Mengen aus, welche korrespondierende jeweilige, regelmäßige Antigen-Spot-Muster ausbilden. Ferner sind Referenz-Spots mit dem gleichen Farbstoff aufgebracht sind, welche eine Referenz-Spot-Menge ausbilden, welche ein regelmäßiges Referenz-Muster ausbildet. Das Referenz-Muster unterscheidet sich von den Antigen-Spot-Mustern hinsichtlich seiner Regelmäßigkeit. Auf Basis einer ersten Bildinformation, welche eine Färbung der Referenz-Spots und der Antigen-Spots durch den gleichen, ersten Farbstoff repräsentiert, und auf Basis einer zweiten Bildinformation, welche eine potentielle Färbung der Antigen-Spots durch einen zweiten Farbstoff nach einer Inkubation repräsentiert wird dann mittels Bildverarbeitung eine Bindung von Antikörpern der biologischen Probe an jeweilige Antigen-Typen bestimmt.

## Beschreibung

Die Erfindung betrifft ein Verfahren zum automatisierten Detektieren von Antikörpern in einer flüssigen biologischen Probe mittels Verwendung eines Antigen-Chips sowie einen entsprechenden Antigen-Chip hierfür.

Die Erfindung betrifft ferner ein Verfahren für eine automatisierte Bildverarbeitung zum Detektieren von Antikörpern in einer flüssigen biologischen Probe.

Die Erfindung betrifft ferner eine Vorrichtung für eine automatisierte Bildverarbeitung zum Detektieren von Antikörpern in einer flüssigen biologischen Probe.

Die Erfindung betrifft ferner eine Datennetzwerkvorrichtung für eine automatisierte Bildverarbeitung zum Detektieren von Antikörpern in einer flüssigen biologischen Probe.

Die Erfindung betrifft ferner ein Computerprogrammprodukt, welche Befehle umfasst, die bei der Ausführung des Programms durch einen Computer diesen veranlassen, ein Verfahren für eine automatisierten Bildverarbeitung zum Detektieren von Antikörpern in einer flüssigen biologischen Probe.

Die Erfindung betrifft ferner ein Datenträgersignal, das das Computerprogrammprodukt überträgt.

Die Erfindung betrifft ferner ein Verfahren zum Herstellen eines Antigen-Chips.

Die Erfindung betrifft ferner einen Objektträger mit einer Mehrzahl von Antigen-Chips.

Die Erfindung betrifft ferner ein Kit zur Verwendung in einem Verfahren zum Detektieren von Antikörpern in einer Probe, aufweisend wenigstens einen Antigen-Chip.

Im Sinne dieser Anmeldung kann eine flüssige, biologische Probe auch als eine flüssige Patientenprobe eines Patienten bezeichnet werden. Ein Beispiel für eine flüssige Patientenprobe ist eine flüssige Patientenprobe, welche Blutsbestandteile aufweist. Insbesondere kann die flüssige Patientenprobe ein Blutserum sein.

Bekannt sind diagnostische Verfahren, bei welchen mittels Antigenen ein Vorhandensein von Antikörpern in einer flüssigen biologischen Probe wie z.B. Blutserum detektiert wird. Hierzu werden Substrate, auch Chips genannt, verwendet, auf deren Oberfläche Antigene in Form von Geltropfen bzw. Spots aufgebracht werden. Die Tropfen bzw. Spots trocknen nach der Aufbringung an der Oberfläche des Substrates an. Um Nachzuweisen, ob Antikörper eines bestimmten Typs in einer flüssigen biologischen Patientenprobe vorhanden sind, werden die Spots zunächst mit der Probe inkubiert, wobei dann abhängig von dem Typ der Antigene und dem Typ der Antikörper es zu einer Bindung von Antikörpern an Antigene kommen kann. Die Antikörper der Probe werden in einem solchen Verfahren mitunter auch als erste Antikörper bezeichnet. Anschließend erfolgt dann eine Inkubation der Spots mit einem Konjugat, welches zweite Antikörper aufweist, die wiederum mit einem Farbstoff markiert sind. Die zweiten Antikörper binden dann an die ersten Antikörper an, so dass dann mittels eines Nachweises einer Färbung der Spots durch den Farbstoff eine Bindung der ersten Antikörper an die Antigene indirekt nachgewiesen werden kann um Aufschluss darüber zu erhalten, ob erste Antikörper eines bestimmten Typs in der Probe enthalten sind bzw. waren. Der Farbstoff kann hierbei beispielsweise ein Fluoreszenzfarbstoff sein.

WO 2012/094427 A1 offenbart Verfahren für Fluoreszenzdetektion von Antikörpern durch Fluoreszenzdetektion. US 2005/0124017 offenbart Fluoreszenzbildgebung zur Detektion von Proteinen, Antikörpern, Drogen oder anderen Liganden in einer Probe, wobei ein Bild abgetastet wird. US 2004/0253640 offenbart ein Microarray mit darauf gedruckten Proteinen, um ein Zielprotein durch Immunofluoreszenz zu detektieren. WO 2017/025954 A1 offenbart einen Antigen-Chip für Immunofluoreszenzdetektion, wobei der Antigen-Chip zur Detektion abgetastet wird. WO 2012/052994 A2 offenbart Microarrays zur Hochdurchsatzcharakterisierung der Immunantwort. WO 2012/037369 A offenbart Antikörperdetektion durch Fluoreszenzdetektion. WO 2004/027379 A offenbart eine Technik von rollenden Kugeln, um primäre Antikörper zu detektieren, welche auf einem Antigen-Microarray gebunden sind. WO 2000/063701 A2 offenbart Microarrays von Polypeptiden, um zum Beispiel Antikörper zu detektieren, wobei Fluoreszenzdetektion verwendet wird und die Microarrays abgetastet werden. WO 2011/101487 A1 offenbart ein Verfahren zur Krankheitsdiagnose über eine simultane Detektion von Antikörpern, welche an synthetische und zelluläre Substrate gebunden sind, wobei die Antikörper durch indirekte Immunofluoreszenz detektiert werden. Ein synthetisches Substrat ist ein Mikropartikel oder eine Kugel, welche mit einem aufgereinigten nativen Antigen oder einem rekombinanten Antigen beschichtet ist, ein Fluoreszenzmikroskop ist mit einer Kamera und mit einem Abtastsystem ausgestattet. EP 2 362 222 offenbart ein Verfahren zur Krankheitsdiagnose, wobei simultan Antikörper detektiert werden, welche an zelluläre oder Gewebesubstrate gebunden sind, oder welche an synthetische Substrate gebunden sind, wie etwa Mikropartikel oder Kugeln, welche mit den spezifischen Antigenen beschichtet sind. Dabei wird Vielfarbfluoreszenzmikroskopie benutzt, um die gebundenen Antikörper zu detektieren, wobei die Substrate und die gebundenen Antikörper verschiedene Fluoreszenzfarben aufweisen.

Bekannt ist es aus dem Stand der Technik, wie z.B. der US 2017 001 60 52, dass Antigen-Spots einen sogenannten ersten Farbstoff aufweisen können, welcher in einem ersten Farbkanal sichtbar wird. Der Farbstoff, mit welchem der zweite Antikörper markiert ist, wird dann als sogenannter zweiter Farbstoff bezeichnet, welcher wiederum in einem zweiten Farbkanal sichtbar wird. Zur Positionserkennung werden hierbei im Stand der Technik alle Spots in Form eines regelmäßigen Gitters entsprechend eines Schachbrettmusters angeordnet, wobei unterschiedliche Spots unterschiedliche Antigene aufweisen. Anhand des ersten Farbstoffs können die Spots dann hinsichtlich der Position bzw. ihrer Lage auf dem Substrat in einem ersten Bild des ersten Farbkanals erkannt werden, da der erste Farbstoff unabhängig von einer Bindung von Antikörpern der Probe mit Antigenen der Spots vorhanden bzw. sichtbar ist. Es können somit in dem ersten Bild des ersten Farbkanals alle Spots, welche den ersten Farbstoff aufweisen, in ihrer Position bzw. der Lage erkannt werden. Es wird dann auf Basis des ersten Bildes ein Raster so gegenüber dem Bild bzw. gegenüber allen Spots ausgerichtet, dass die einzelnen Felder des Rasters jeweils genau einen Spot erfassen. Dieses starre Raster bedarf also in allen Feldern einer genauen Ausrichtung gegenüber dem Spots des einen, starren Musters. Ist das Raster einmal an das erste Bild angepasst, so kann anhand der einzelnen Rasterposition bzw. der einzelnen Feldposition innerhalb des Rasters dann auf Basis einer zugehörigen Dateninformation jeweils festgestellt bzw. angegeben werden, welcher bestimmte, erkannte Spot aus welchem bestimmten Feld welches genaue Antigen trägt. Es muss also ein bestimmter Antigen-Spot mit einem bestimmten Antigen immer genau in einem bestimmten Feld des Rasters erkannt werden, um eine solche Antigenzuordnung fehlerfrei vornehmen zu können. Eine in dem Bild des ersten Farbkanals gewonnene Positions-Information für einen Spot kann dann bezogen auf ein Bild des zweiten Farbkanals genutzt werden, um festzustellen, ob eine Färbung durch den zweiten Farbstoff an eben jener Position eine Bindung von ersten Antikörpern an einen bestimmten Antigen-Typ indiziert.

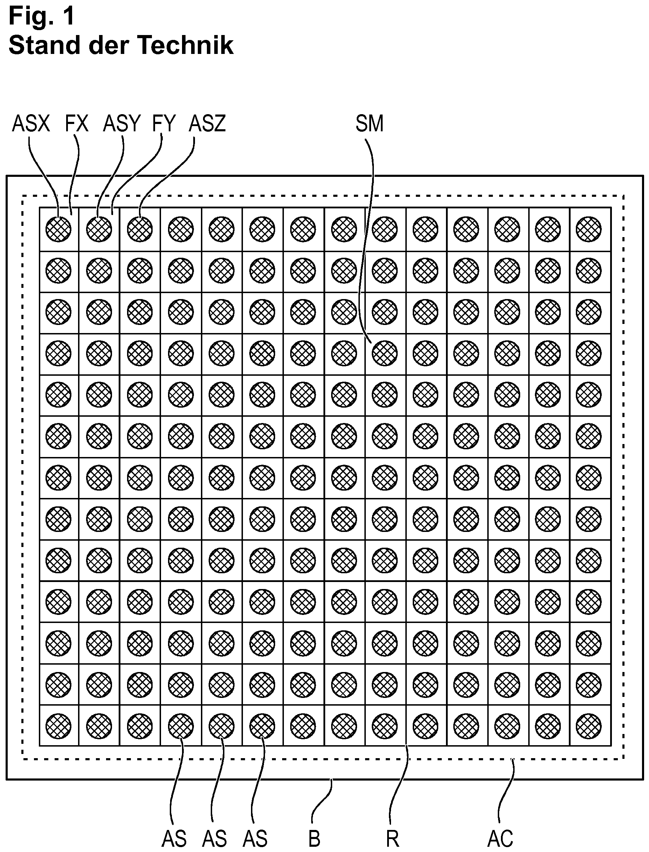

In der Fig. 1 ist ein aus dem Stand der Technik bekannter Antigen-Chip AC mit Antigen-Spots AS für einen aufgenommenen Bildbereich B für einen ersten Farbkanal bzw. ersten Farbstoff beispielhaft dargestellt. Ein zu erzeugendes bzw. zu erkennendes Raster R ist ebenfalls dargestellt. Der Chip AC trägt ein Spot-Muster SM. Es sei angenommen, dass der Antigen-Spot ASX ein anderes Antigen trägt als der Antigen-Spot ASY. Der linke, obere Antigen-Spot ASX muss daher in dem linken oberen Feld FX des Rasters liegen, sodass eine Zuordnung zu einem bestimmten Antigen korrekt erfolgen kann.

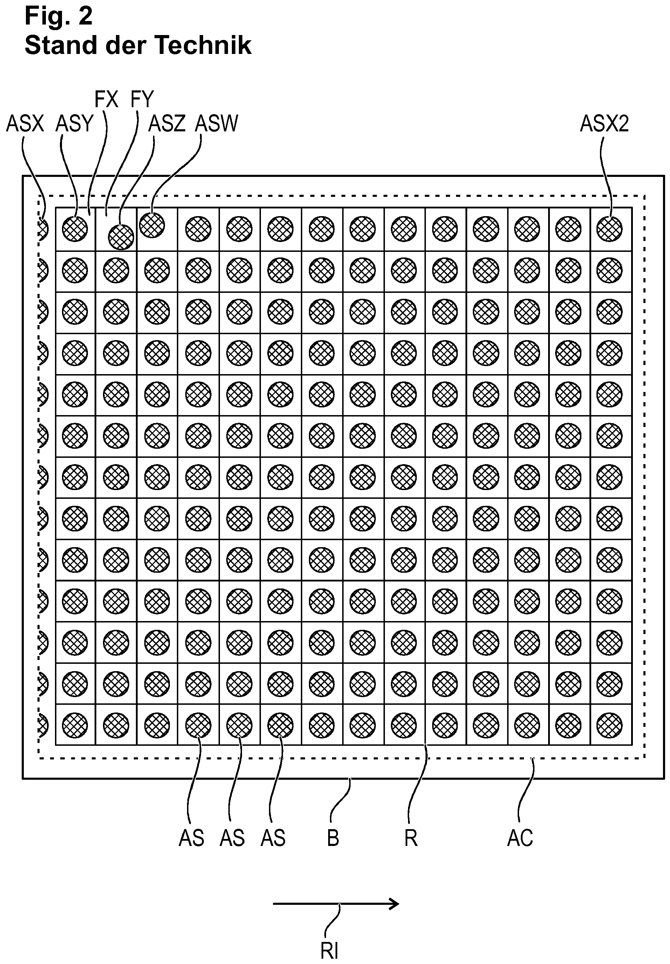

Die Fig. 2 zeigt eine Konfiguration, bei welcher auf dem Substrat des Chips AC keine genaue bzw. keine exakte örtliche Anordnung der Spots AS erfolgte. Daher ist der Spot ASX nur teilweise auf dem Chip AC und in dem Bildbereich B vorhanden, so dass das Raster R zwar an sich detektiert werden könnte, es aber zu einer Fehlausrichtung der Felder FX, FY gegenüber den Spots ASX, ASY kommen kann. Daher wäre durch eine Zuordnung eines Antigentyps zu dem Spot ASY auf Basis des dem Spot ASY zugeordneten Rasterfeldes FX fehlerhaft und dann eben auch eine Bindungsdetektion in Bezug auf eine Bindung von Antikörpern der biologischen Probe an ein bestimmtes Antigen. Diese Fehldetektion läge insbesondere dann vor, wenn das gesamte Spot-Muster SM im Rahmen eines Produktionsprozesses repititiv in der Richtung RI wiederholt werden würde, so dass ein Antigen-Spot ASX2 vorhanden wäre, welcher das gleiche Antigen wie der Spot ASX tragen würde. Es müsste also im Rahmen eines Produktionsprozesses das gesamte Spot-Muster SM auf dem Chip AC sehr genau positioniert werden, um eine Fehlzuordnung von Antigen-Typen zu Antigen-Spots ASX, ASY, ASZ zu vermeiden. Der Stand der Technik erfordert also eine hohe Präzision im Zuge einer Herstellung des Substrates bzw. Antige-Chips, welcher unterschiedliche Antigen-Spots mit unterschiedlichen Antigen-Typen trägt. Der aus dem Stand der Technik bekannte Antigen-Chip ist ferner sensitiv für eine Abweichung einer Position von Antigen-Spots wie z.B. der Spots ASZ, ASW des Musters SM.

Vorgeschlagen wird daher ein erfindungsgemäßes Verfahren zum automatisierten Detektieren von Antikörpern in einer flüssigen biologischen Probe mittels Verwendung eines Antigen-Chips sowie ein entsprechender erfindungsgemäßer Antigen-Chip.

Der erfindungsgemäße Antigen-Chip weist eine ebene Substratoberfläche mit darauf aufgebrachten, zueinander beabstandeten Antigen-Spots auf, welche einen gleichen, gemeinsamen Farbstoff aufweisen. Dieser Farbstoff kann auch als ein erster Farbstoff bezeichnet werden. Die Antigen-Spots bilden hierbei jeweilige Antigen-Spot-Mengen aus, welche jeweilige, regelmäßige Antigen-Spot-Muster ausbilden, wobei Antigen-Spots einer gleichen Antigen-Spot-Menge einen gleichen, gemeinsamen Antigentyp aufweisen. Hierbei weisen zwei oder mehr der Antigen-Spot-Mengen unterschiedliche Antigen-Typen auf. Der erfindungsgemäße Antigen-Chip ist dadurch gekennzeichnet, dass auf der Substratoberfläche ferner Referenz-Spots aufgebracht sind, welche ebenfalls den gleichen, gemeinsamen Farbstoff aufweisen. Die Referenz-Spots bilden eine Referenz-Spot-Menge aus, die wiederum ein regelmäßiges Referenz-Muster ausbildet, insbesondere entlang einer Anordnungsrichtung, in welcher die Muster aufeinander abfolgen. Das Referenz-Muster unterscheidet sich hinsichtlich seiner Regelmäßigkeit von den Antigen-Spot-Mustern bzw. von den Regelmäßigkeiten der Antigen-Spot-Muster.

Der erfindungsgemäße Antigen-Chip findet dann Anwendung in dem erfindungsgemäßen Verfahren zum automatisierten Detektieren von Antikörpern in einer flüssigen biologischen Probe. Der gleiche, gemeinsame Farbstoff ist hierbei eben auch als ein erster Farbstoff zu bezeichnen. Im Zuge des Verfahrens werden die Spots der Antigen-Chip zunächst mit der biologischen Probe inkubiert. Ferner werden die Spots mit einem Konjugat inkubiert, welches einen mit einem zweiten Farbstoff markierten sekundären Antikörper aufweist. Hierdurch können sich an bestimmten Antigen-Spots die zuvor beschriebenen Bindungen der sekundären Antikörper und somit auch des zweiten Farbstoffs ergeben, abhängig von dem Typ des Antigens eines Antigen-Spots und abhängig von in der Probe enthaltener primärer Antikörper. Im Zuge des Verfahrens wird dann eine erste Bildinformation erfasst oder bereitgestellt, welche eine Färbung der Referenz-Spots und der Antigen-Spots durch den ersten Farbstoff repräsentiert. Auf Basis der wenigstens einen ersten Bildinformation erfolgt dann ein Detektieren des Referenz-Musters sowie einer zu dem Referenz-Muster zugehörigen Referenz-Position. Ferner erfolgt dann auf Basis der wenigstens einen ersten Bildinformation ein Detektieren der jeweiligen Antigen-Spot-Muster sowie jeweiliger zugehöriger weiterer Positionen. Ferner erfolgt auf Basis der detektierten Referenz-Position und der detektierten weiteren Positionen ein Erstellen einer Zuordnungs-Information, welche eine Zuordnung der jeweiligen Antigen-Spot-Muster zu jeweiligen Antigen-Typen indiziert. Schließlich erfolgt ein Erfassen oder Bereitstellen einer zweiten Bildinformation, welche eine potentielle Färbung der Antigen-Spots durch den zweiten Farbstoff repräsentiert. Abschließend erfolgt, auf Basis der zweiten Bildinformation und der Zuordnungs-Information, ein Bestimmen jeweiliger Bindungsmaße, welche jeweilige Bindungen von Antikörpern der biologischen Probe an jeweilige Antigen-Typen indizieren.

Insbesondere sind das Referenz-Muster und die Antigenmuster entlang einer Anordnungsrichtung der Muster angeordnet, wobei ferner insbesondere das Referenz-Muster und die Antigenmuster jeweilige Ausbreitungsrichtungen aufweisen, welche im Wesentlichen senkrecht zu der Anordnungsrichtung der Muster verlaufen.

Das Referenz-Muster und die Antigen-Spot-Muster weisen vorzugsweise jeweilige Ausbreitungsrichtungen auf, welche senkrecht zu einer Anordnungsrichtung der Muster verläuft. Die Ausbreitungsrichtungen der Muster verlaufen vorzugsweise im Wesentlichen parallel zueinander.

Auf dem erfindungsgemäßen Antigen-Chip weisen die Antigen-Spots sowie die Referenz-Spots den gleichen, gemeinsamen Farbstoff auf, welcher in einem ersten Farbkanal sichtbar ist, welches vorzugsweise ein sogenannter Rotkanal ist.

Der erfindungsgemäße Antigen-Chip und das erfindungsgemäße Verfahren erreichen einen oder mehrere der nun im Folgenden genannten Vorteile, wofür im Weiteren detaillierte Ausführungen folgen.

Dadurch, dass der Antigen-Chip Referenz-Spots aufweist, welche ein eigenes regelmäßiges Referenz-Muster ausbilden, das sich hinsichtlich seiner Regelmäßigkeit von den Antigen-Spot-Mustern der Antigen-Spots unterscheidet, ist es möglich, eine Position bzw. Lage des Referenz-Musters in dem ersten Farbkanal bzw. der ersten Bildinformation zu detektieren. Da die Antigen-Spot-Muster sich in ihrer Art des Musters bzw. ihrer Regelmäßigkeit von dem Referenz-Muster unterscheiden, können die Antigen-Spot-Muster separat hinsichtlich ihrer Position bzw. Lager auf der Substratoberfläche detektiert werden. Es kann dann eben festgestellt werden, welches der Antigen-Spot-Muster welchen Antigen-Typen aufweist, da die Referenz-Position des Referenz-Musters in Beziehung zu weiteren Positionen der weiteren Antigen-Spot-Muster anzeigt, ab wo bzw. ab welcher Position eine Abfolge der Antigen-Spot-Muster in einer Anordnungsrichtung der Muster auf der Substratoberfläche abfolgen. Es muss also ein bestimmtes Antigen-Spot-Muster mit einem bestimmten Antigen-Typen nicht an einer ganz bestimmten Stelle des Bildes bzw. der Substratoberfläche angeordnet sein. Vielmehr reicht es, dass ein Antigen-Spot-Muster in einer bestimmten Position gegenüber der Referenz-Position des Referenz-Musters entlang einer Anordnungsrichtung der Muster angeordnet ist. Ein Erstellen einer Zuordnungs-Information, welche eine Zuordnung jeweiliger Antigen-Spot-Muster zu jeweiligen Antigen-Typen indiziert, kann dann beispielsweise dadurch erfolgen, dass ein Datensatz bereitgestellt wird, welcher eine solche Zuordnung in Abhängigkeit einer Position des Antigen-Spot-Musters zu einer Position des Referenz-Musters anzeigt. Hierdurch wird das erfindungsgemäße Verfahren besonders robust, da es lediglich erforderlich ist, die Antigen-Spots einer bestimmten Antigen-Spotmenge als ein Antigen-Spot-Muster so anzuordnen, dass das entsprechende Muster sich von anderen Mustern beispielsweise in seiner Position hinreichend absetzt. Bei der Lösung nach dem Stand der Technik bilden die Antigen-Spots keine eigenen regelmäßigen Muster aus, sondern sind in einer Art Schachbrettverfahren an unterschiedlichsten Positionen angeordnet, wobei eine Zuordnung eines bestimmten Antigentyps zu Spots eben sehr sensitiv für die einzelne Ausrichtung der Spots auf der Substratoberfläche bzw. einem Bild ist.

Der erfindungsgemäße Antigen-Chip ist besonders deshalb ferner vorteilhaft, da, wie zuvor beschrieben, es lediglich auf die Abfolge des Referenz-Musters und der Antigen-Spot-Muster in der Anordnungsrichtung bzw. entlang der Anordnungsrichtung der Muster auf der Substratoberfläche ankommt, so dass das Referenz-Muster und die weiteren Antigen-Spot-Muster lediglich in einer geordneten Abfolge aufeinander entlang der Anordnungsrichtung auftreten müssen. Dieses hat besondere Vorteile im Zuge eines Produktionsprozesses, da bei einem Aufbringen der Gesamtheit der Muster in periodischer, sich wiederholender bzw. repetitiver Weise, so dass also die Gesamtheit der Muster periodisch aufeinander wiederholt wird, der Substratträger anschließend nicht exakt an jener Stelle aufgetrennt bzw. fragmentiert werden muss, an welcher sich die Gesamtheit der Muster wiederholt, um mehrere Antigen-Chips mit jeweils der Gesamtheit der Muster herzustellen. Es ist lediglich erforderlich, dass alle erwünschten Antigen-Spotmengen bzw. Antigen-Spot-Muster und das Referenz-Muster bzw. die Referenz-Spotmenge jeweils wenigstens einmal auf dem zu produzierenden Antigen-Chip vorkommt. Eine solche Gesamtheit der Muster bzw. eine solche Abfolge dieser Antigen-Spot-Muster und des Referenz-Musters ergibt sich üblicherweise über eine vorbestimmte Distanz entlang der Anordnungsrichtung der Muster auf dem Substratträger. Es muss eben bei dem erfindungsgemäßen Antigen-Chip diese Distanz nicht exakt eingehalten werden, sondern es ist eben ausreichend, wenn die Wiederholungs-Distanz der Gesamtheit der Muster entlang der Anordnungsrichtung der Muster bei der Herstellung des Antigen-Chips lediglich nicht unterschritten wird. Es kann eben beispielsweise die Substratoberfläche auch nach einer solchen Distanz entlang der Anordnungsrichtung aufgetrennt bzw. fragmentiert werden, welche größer als die Wiederholungs-Distanz der Gesamtheit der Muster in der Anordnungsrichtung ist, innerhalb welcher alle unterschiedlichen Muster wenigstens einmal enthalten sind. Es ist eben ausreichend, wenn die Distanz zwischen zwei Fragmentierungsstellen größer als die Wiederholungs-Distanz der Gesamtheit der Muster ist und ferner auch kleiner als die zweifache Wiederholungs-Distanz der Gesamtheit der Muster. Das Auftrennen bzw. Fragmentieren erfolgt hierbei insbesondere in einer Richtung senkrecht zu der Anordnungsrichtung der Muster.

Der erfindungsgemäße Antigen-Chip und das erfindungsgemäße Verfahren sind ferner vorteilhaft, da eine leichte Fehlausrichtung einzelner Spots innerhalb eines bestimmten Musters ein Effekt sind, auf welchen das erfindungsgemäße Verfahren weniger sensitiv reagiert als die Lösung nach dem Stand der Technik, bei welchem alle Spots in ein klares definiertes und starres Muster mit gleichen Distanzen zwischen allen benachbarten Spots hineinpassen müssen. Bei dem erfindungsgemäßen Antigen-Chip ist es eben möglich, dass bei der Anordnung der Muster entlang einer Anordnungsrichtung aufeinander abfolgend auf der Substratoberfläche eine Beabstandung der Muster zueinander zwischen unterschiedlichen Mustern auch unterschiedlich ausgestaltet sein kann. Werden die Muster in dem erfindungsgemäßen Verfahren beispielsweise dadurch erkannt bzw. detektiert, dass die Regelmäßigkeit eines Musters darin besteht, dass die Spots einer entsprechenden Spotmenge bzw. eines betreffenden Spot-Musters im Mittel einen bestimmten Abstand zueinander haben, so kann ein nächstes, benachbartes Muster zu dem vorherigen Muster einen Nachbar-Abstand aufweisen, welcher gegenüber einem anderen Nachbar-Abstand zwischen anderen Mustern unterschiedlich sein kann. Dennoch kann das erfindungsgemäße Verfahren durch eine Bildanalyse des erfindungsgemäßen Antigen-Chips immer noch entsprechende Positionen der Muster robust erkennen und die jeweiligen Spots jeweiligen Antigen-Typen zuordnen.

Bezogen auf das Referenz-Muster sowie die Antigenmuster lässt sich ferner sagen, dass die Regelmäßigkeit des Referenz-Musters sich von der Regelmäßigkeit der Antigen-Spot-Muster dadurch unterscheidet, dass die Muster, also das Referenz-Muster sowohl als auch die Antigen-Spot-Muster, einen gemeinsamen Regelmäßigkeitsparameter haben, dessen Wert sich bei dem Referenz-Muster von den Werten dieses Regelmäßigkeitsparameters der Antigen-Spot-Muster unterscheidet.

Vorzugsweise ist der gleiche, gemeinsame Farbstoff ein Fluoreszenzfarbstoff, welcher auch als erster Fluoreszenzfarbstoff bezeichnet werden kann. Alternativ ist der gleiche, gemeinsame Farbstoff ein chromogenes Substrat, welches zum Zwecke einer chromogenen Verfärbung eine Bindung mit einem Enzym eingehen kann, welches wiederum in einem Konjugat enthalten ist, mittels dessen die Spots des Antigen-Chips inkubiert werden können.

Vorzugsweise ist der zweite Farbstoff ein Fluoreszenzfarbstoff, welcher als zweiter Fluoreszenzfarbstoff bezeichnet werden kann.

In einem Verfahrensschritt können die jeweiligen Fluoreszenzfarbstoffe durch Einstrahlung von Anregungslicht jeweiliger unterschiedlicher Anregungswellenlängen oder einer gleichen Anregungswellenlänge zur Emission jeweiliger Fluoreszenzstrahlung jeweiliger, unterschiedlicher Fluoreszenzwellenlängen angeregt werden. Hierbei können sich die Anregungslicht-Wellenlängen für die beiden Fluoreszenzfarbstoffe überlappen bzw. auch identisch sein.

Vorzugsweise ist der Antigen-Chip dadurch gekennzeichnet, dass der gleiche, gemeinsame Farbstoff ein Fluoreszenzfarbstoff ist.

Vorzugsweise ist der Antigen-Chip dadurch gekennzeichnet, dass der zweite Farbstoff ein Fluoreszenzfarbstoff ist.

Gemäß einer Ausführungsform ist der Antigen-Chip dadurch gekennzeichnet, dass sich das Referenz-Muster hinsichtlich seiner Regelmäßigkeit von den Antigen-Spot-Mustern durch eines oder mehrere der folgenden Kriterien unterscheidet:
a) innerhalb des Referenz-Musters und innerhalb der Antigen-Spot-Muster folgen die jeweils zugehörigen Spots in jeweils regelmäßigen Abständen aufeinander, wobei der regelmäßige Abstand der Referenz-Spots des Referenz-Musters sich von den regelmäßigen Abständen der Antigen-Spots der Antigen-Spot-Muster unterscheidet; der regelmäßige Abstand ist hierbei der Regelmäßigkeitsparamteer eines Musters;
b) das Referenz-Muster weist zu einem unmittelbar benachbarten Muster einen solchen Nachbar-Abstand auf, welcher sich von den anderen Nachbar-Abständen der Antigen-Spot-Muster unterscheidet; der Nachbar-Abstand zu einem benachbarten Muster in Anordnungsrichtung der Muster ist hierbei der der Regelmäßigkeitsparameter eines Musters;
c) innerhalb des Referenz-Musters und innerhalb der Antigen-Spot-Muster weisen die jeweils zugehörigen Spots eine jeweilige, gleiche Größe auf, so dass die Größe der Referenz-Spots des Referenz-Musters sich von den Größen der Antigen-Spots der Antigen-Spot-Muster unterscheidet; die gleiche Größe der Spots eines Musters ist hierbei der Regelmäßigkeitsparameter eines Musters.

Gemäß einer Ausführungsform ist der Antigen-Chip dadurch gekennzeichnet, dass die Referenz-Spots der Referenz-Spot-Menge ein Referenz-Linienmuster ausbilden und dass ferner die jeweiligen Antigen-Spots der jeweiligen Antigen-Spot-Mengen jeweilige Antigen-Linienmuster ausbilden.

Gemäß einer Ausführungsform ist der Antigen-Chip dadurch gekennzeichnet, dass das Referenz-Linienmuster sich hinsichtlich seiner Regelmäßigkeit von den AntigenLinienmustern durch eines oder mehrere der folgenden Kriterien unterscheidet:
a) innerhalb des Referenz-Linienmusters und innerhalb der Antigen-Linienmuster folgen die jeweils zugehörigen Spots in jeweils regelmäßigen Abständen aufeinander, wobei der regelmäßige Abstand der Referenz-Spots des Referenz-Linienmusters sich von den regelmäßigen Abständen der Antigen-Spots der Antigen-Linienmuster unterscheidet;
b) das Referenz-Linienmuster weist zu einem unmittelbar benachbarten Linienmuster einen Nachbar-Abstand auf, welcher sich von den anderen Nachbar-Abständen der ersten Linienmuster unterscheidet;
c) innerhalb des Referenz-Linienmusters und innerhalb der Antigen-Linienmuster weisen die jeweils zugehörigen Spots eine jeweilige, gleiche Größe auf, so dass die Größe der Referenz-Spots des Referenz-Linienmusters sich von den Größen der Antigen-Spots der Antigen-Linienmuster unterscheidet;

Vorzugsweise unterscheidet sich der Abstand der Antigen-Spots zueinander innerhalb eines Musters für wenigstens eines der Muster von einem Beabstandungsabstand bzw. Nachbar-Abstand des Muster zu einem benachbarten Muster in der Anordnungsrichtung der Muster.

Vorzugsweise ist der Antigen-Chip dadurch gekennzeichnet, dass die Referenz-Spots ferner ein Antigen aufweisen.

Vorzugsweise ist der Antigen-Chip dadurch gekennzeichnet, dass die Referenz-Spots ferner einen Antikörper, insbesondere IgG, aufweisen.

Im Zuge des erfindungsgemäßen Verfahrens zum automatisierten Detektieren von Antikörpern in einer flüssigen biologischen Probe erfolgt vorzugsweise eine Ausgabe der Bindungsmaße, insbesondere in Form eines Datenelementes, welche insbesondere über eine Datenschnittstelle ausgegeben wird.

Vorgeschlagen wird ferner ein Verfahren für eine automatisierte Bildverarbeitung zum Detektieren von Antikörpern in einer flüssigen biologischen Probe, aufweisend die Schritte
- Bereitstellen oder Erfassen einer ersten Bildinformation, welche eine Färbung von Referenz-Spots und von Antigen-Spots eines erfindungsgemäßen Antigen-Chips nach einer der hier vorgeschlagenen Ausführungsformen durch einen ersten Farbstoff repräsentiert,
- Detektieren des Referenz-Musters sowie einer zugehörigen Referenz-Position auf Basis der wenigstens einen ersten Bildinformation,
- Detektieren der jeweiligen Antigen-Spot-Muster sowie jeweiliger zugehöriger weiterer Positionen auf Basis der wenigstens einen ersten Bildinformation,
- auf Basis der detektierten Referenz-Position und der detektierten weiteren Positionen, Erstellen einer Zuordnungs-Information, welche eine Zuordnung der jeweiligen Antigen-Spot-Muster zu jeweiligen Antigen-Typen indiziert,
- Bereitstellen oder Erfassen einer zweiten Bildinformation, welche eine potentielle Färbung der Antigen-Spots des erfindungsgemäßen Antigen-Chips nach einer der hier vorgeschlagenen Ausführungsformen durch einen zweiten Farbstoff nach Inkubieren des Antigen-Chips mit der biologische Probe und mit einem Konjugat, welches einen mit dem zweiten Farbstoff markierten sekundären Antikörper aufweist, repräsentiert,
- auf Basis der zweiten Bildinformation, Bestimmen jeweiliger Bindungsmaße, welche jeweilige Bindungen bzw. Bindungsgrade von Antikörpern der biologischen Probe an jeweilige Antigen-Typen indizieren.

Im Zuge des Verfahrens für eine automatisierte Bildverarbeitung zum Detektieren von Antikörpern einer flüssigen biologischen Probe wird vorzugsweise die erste Bildinformation über wenigstens eine Datenschnittstelle entgegengenommen wird und mittels wenigstens einer Speichereinheit bereitgestellt wird, und es wird ferner vorzugsweise die zweite Bildinformation über die Datenschnittstelle entgegengenommen und mittels der Speichereinheit bereitgestellt.

In dem Fall, dass der Antigen-Chip die zuvor erwähnten ersten und zweiten Referenz-Spots zur Ausbildung des ersten regelmäßigen Referenz-Musters und des zweiten regelmäßigen Referenz-Musters aufweist, erfolgen vorzugsweise in dem Verfahren zum automatisierten Detektieren von Antikörpern in einer flüssigen biologischen Probe sowie in dem Verfahren für eine automatisierte Bildverarbeitung zum Detektieren von Antikörpern in einer flüssigen biologischen Probe weitere Schritte. Diese weiteren, vorzugsweise durchzuführenden Schritte sind:
- Detektieren des zweiten Referenz-Musters sowie einer zweiten zugehörigen Referenz-Position auf Basis der ersten Bildinformationen,
- Erstellen der Zuordnungs-Informationen, welche die Zuordnung der jeweiligen Antigen-Spot-Muster zu den jeweiligen Antigen-Typen indiziert, auf Basis der detektierten ersten Referenz-Position, der detektierten zweiten Referenz-Position und der detektierten weiteren Positionen.

Im Zuge des Verfahrens zum automatisierten Detektieren von Antikörpern sowie des Verfahrens für eine automatisierte Bildverarbeitung erfolgt die Zuordnung der jeweiligen detektierten Antigen-Spot-Muster zu jeweiligen Antigen-Typen mittels weiterer vorzugsweise durchzuführender Schritte:
- Bestimmen einer räumlichen Abfolge der Referenz-Position und der weiteren Positionen
- Bereitstellen eines Abfolge-Datensatzes, welcher eine Abfolge von Antigen-Typen indiziert,
- Erstellen der Zuordnungs-Information, welche die Zuordnung der jeweiligen Antigen-Spot-Muster zu den jeweiligen Antigen-Typen indiziert, auf Basis der detektierten Referenz-Position, der detektierten weiteren Positionen und des Abfolge-Datensatzes.

Vorzugsweise erfolgen in einem oder beiden der zuvor genannten Verfahren weitere Schritte:
- Erkennen mehrerer potentieller Muster auf Basis der wenigstens einen ersten Bildinformation,
- Detektieren jeweiliger Spot-Abstände Spots innerhalb der jeweiligen erkannten potentiellen Muster bzw. für die jeweils erkannten, potentiellen Muster,
- Auswählen eines der erkannten Muster als das Referenz-Muster auf Basis der detektierten Spot-Abstände, vorzugsweise anhand eines höchsten oder eines geringsten Spot-Abstandes.

Vorzugsweise sind die Muster der Spots auf dem Antigen-Chip derart ausgestaltet, dass eine Unterscheidung des Referenz-Musters gegenüber den Antigen-Spot-Mustern dadurch gegeben ist, dass das Referenz-Muster zu einem unmittelbar benachbarten Muster, insbesondere entlang einer Anordnungsrichtung der Muster, einen solchen Nachbar-Abstand aufweist, welcher auch als Musterabstand bezeichnet werden kann, welcher sich von den anderen Nachbarabständen bzw. Musterabständen der Antigen-Spot-Muster unterscheidet. Es erfolgen dann vorzugsweise im Zuge eines oder beider der zuvor genannten Verfahren weitere vorzugsweise durchgeführte Schritte:
- Erkennen mehrerer Muster auf Basis der wenigstens einen ersten Bildinformation,
- Detektieren jeweiliger Nachbar-Abstände der erkannten Muster, insbesondere entlang einer Anordnungsrichtung der Muster, zu ihren - insbesondere direkt oder unmittelbar-jeweils nächsten benachbarten Mustern,
- Auswählen eines der erkannten Muster als das Referenz-Muster auf Basis der detektierten Nachbar-Abstände.

In dem Fall, dass der Antigen-Chip derart ausgeführt ist, dass die Spots innerhalb des Referenz-Musters und auch innerhalb der Antigen-Spot-Muster in jeweils regelmäßigen - vorzugsweise äquidistanten- Abständen aufeinanderfolgen, wobei der regelmäßige Abstand der Referenz-Spots des Referenz-Musters sich von den regelmäßigen Abständen der Antigen-Spots der Antigen-Spot-Muster unterscheidet, können weitere vorzugsweise Schritte durchgeführt werden im Zuge eines oder beider der genannten Verfahren:
- Erkennen mehrerer Muster auf Basis der wenigstens einen ersten Bildinformation,
- Detektieren jeweiliger Spot-Größen für die jeweiligen erkannten Muster,
- Auswahl eines der erkannten Muster als das Referenz-Muster Muster auf Basis der detektierten Spot-Größen.

In dem Fall, dass der erste Farbstoff ein Fluoreszenzfarbstoff ist können in einem oder beiden der zuvor genannten Verfahren weitere Schritte vorzugsweise durchgeführt werden:
- Beleuchten des Antigen-Chips mit erstem Anregungslichteines eines ersten Wellenlängenbereiches zum Anregen einer Emission von erster Fluoreszenzstrahlung durch den ersten Farbstoff,

Hierbei repräsentiert die erste Bildinformation vorzugsweise eine Färbung der Referenz-Spots und der Antigen-Spots des Antigen-Chips durch die erste Fluoreszenzstrahlung.

In dem Fall, dass der zweite Farbstoff ein Fluoreszenzfarbstoff ist, können in einem oder beiden der zuvor genannten Verfahren weitere Schritte vorzugsweise durchgeführt werden:
- Beleuchten des Antigen-Chips mit zweitem Anregungslicht eines zweiten Wellenlängenbereiches zum Anregen einer Emission von zweiter Fluoreszenzstrahlung durch den zweiten Farbstoff.

Hierbei repräsentiert die zweite Bildinformation vorzugsweise eine potentielle Färbung der Antigen-Spots des Antigen-Chips durch die erste Fluoreszenzstrahlung repräsentiert.

Vorzugsweise weisen das erste Anregungslicht und das zweite Anregungslicht eine gleiche Anregungswellenlänge auf.

Vorgeschlagen wird ferner eine Vorrichtung zum automatisierten Detektieren von Antikörpern in einer Probe, welche wenigstens eine Bilderfassungseinheit aufweist. Die Bilderfassungseinheit ist ausgebildet zum Erfassen einer ersten Bildinformation, welche eine Färbung von Referenz-Spots und von Antigen-Spots eines erfindungsgemäßen Antigen-Chips nach einer der hier beschriebenen Ausführungsformen durch einen ersten Farbstoff repräsentiert. Die Bilderfassungseinheit ist ferner ausgebildet zum Erfassen einer zweiten Bildinformation, welche eine potentielle Färbung der Antigen-Spots des erfindungsgemäßen Antigen-Chips nach einer der hier beschriebenen Ausführungsformen durch einen zweiten Farbstoff nach Inkubieren des Antigen-Chips mit der biologische Probe und mit einem Konjugat, welches einen mit dem zweiten Farbstoff markierten sekundären Antikörper aufweist, repräsentiert. Die Vorrichtung weist ferner wenigstens eine Recheneinheit auf, welche ausgebildet ist,
- auf Basis der wenigstens einen ersten Bildinformation das Referenz-Muster sowie eine zugehörige Referenz-Position zu detektieren,
- auf Basis der wenigstens einen ersten Bildinformation die jeweiligen Antigen-Spot-Muster sowie jeweilige zugehörige weitere Positionen zu detektieren,
- auf Basis der detektierten Referenz-Position und der detektierten weiteren Positionen eine Zuordnungs-Information zu erstellen, welche eine Zuordnung der jeweiligen Antigen-Spot-Muster zu jeweiligen Antigen-Typen indiziert,
- sowie auf Basis der zweiten Bildinformation jeweilige Bindungsmaße zu bestimmen, welche jeweilige Bindungen bzw. Bindungsgrade von Antikörpern der biologischen Probe an jeweilige Antigen-Typen indizieren.

Die Vorrichtung weist ferner wenigstens eine Beleuchtungseinheit zum Aussenden von Anregungslicht zum Anregen einer Emission von erster Fluoreszenzstrahlung eines ersten Wellenlängenbereiches durch die ersten Farbstoffe auf.

Die Vorrichtung weist ferner wenigstens eine Beleuchtungseinheit zum Aussenden von Anregungslicht zum Anregen einer Emission von zweiter Fluoreszenzstrahlung eines zweiten Wellenlängenbereiches durch den zweiten Farbstoff auf.

Die vorgeschlagene Vorrichtung zum automatisierten Detektieren von Antikörpern einer Probe weist vorzugsweise wenigstens eine Recheneinheit auf, welche ferner ausgebildet ist weitere vorzugsweise Schritte durchzuführen, welche zuvor unter Bezug auf das Verfahren zum automatisierten Detektieren von Antikörpern und/oder das Verfahren für eine automatisierte Bildverarbeitung offenbart bzw. beschrieben wurden.

Vorzugsweise weist die Vorrichtung zum automatisierten Detektieren eine Datenschnittstelle auf, über welche mittels eines Datenelementes oder mehrerer Datenelemente eine Information oder unterschiedliche Informationen bereitgestellt werden können. Es kann sich hierbei um eine oder mehrere der folgenden Informationen handeln:
- eine Information bezogen auf die zuvor erwähnten jeweiligen Bindungsmaße,
- eine Information bezogen auf Positionen von detektierten Mustern,
- eine Information bezogen auf Positionen einzelner Spots,
- eine Information bezogen auf eine Zuordnung von Antigen-Typen zu Antigen-Spot-Mengen oder Antigen-Spot-Mustern.

Vorgeschlagen wird ferner eine Datennetzwerkvorrichtung zur automatisierten Bildauswertung zum Detektieren von Antikörpern in einer Probe. Die Datennetzwerkvorrichtung ist vorzugsweise ein sogenanntes Cloud System. Die Datennetzwerkvorrichtung weist wenigstens eine Datenschnittstelle auf, welche ausgebildet ist zum Entgegennehmen einer ersten Bildinformation, welche eine Färbung von Referenz-Spots und von Antigen-Spots eines erfindungsgemäßen Antigen-Chips nach einer der hier vorgeschlagenen Ausführungsformen durch einen ersten Farbstoff repräsentiert. Die Datenschnittstelle ist ferner ausgebildet zum Entgegennehmen einer zweiten Bildinformation, welche eine potentielle Färbung der Antigen-Spots des erfindungsgemäßen Antigen-Chips nach einer der hier vorgeschlagenen Ausführungsformen durch einen zweiten Farbstoff nach Inkubieren des Antigen-Chips mit der biologische Probe und mit einem Konjugat, welches einen mit dem zweiten Farbstoff markierten sekundären Antikörper aufweist, repräsentiert.

Die Datennetzwerkvorrichtung weist ferner wenigstens eine Recheneinheit auf, welche ausgebildet ist,
- auf Basis der wenigstens einen ersten Bildinformation das Referenz-Muster sowie eine zugehörige Referenz-Position zu detektieren,
- auf Basis der wenigstens einen ersten Bildinformation die jeweiligen Antigen-Spot-Muster sowie jeweilige zugehörige weitere Positionen zu detektieren,
- auf Basis der detektierten Referenz-Position und der detektierten weiteren Positionen eine Zuordnungs-Information zu erstellen, welche eine Zuordnung der jeweiligen Antigen-Spot-Muster zu jeweiligen Antigen-Typen indiziert,
- sowie auf Basis der zweiten Bildinformation jeweilige Bindungsmaße zu bestimmen, welche jeweilige Bindungen bzw. Bindungsgrade von Antikörpern der biologischen Probe an jeweilige Antigen-Typen indizieren.

Vorgeschlagen wird ferner ein Computerprogrammprodukt, welches Befehle umfasst, die bei der Ausführung des Programms durch einen Computer diesen veranlassen, das vorgeschlagene Verfahren für eine automatisierte Bildverarbeitung gemäß einer der hier vorgeschlagenen Ausführungsformen durchzuführen.

Vorgeschlagen wird ferner ein Datenträgersignal, das das Computerprogrammprodukt überträgt. Das Datenträgersignal kann beispielsweise in Form von Datenpaketen, insbesondere IP-Paketen, bereitgestellt werden. Insbesondere ist das Datensignal ein Download-Datensignal zur Übertragung einer Software, insbesondere einer Software-Applikation.

Vorgeschlagen wird ferner ein Objektträger, welcher eine Vielzahl von erfindungsgemäßen Antigen-Chips nach einer der hier beschriebenen Ausführungsformen aufweist.

Vorgeschlagen wird ferner ein Verfahren zur Herstellung eines Antigen-Chips zur Immunodiagnostik aufweisend die Schritte
- Bereitstellen eines Substrats mit einer ebenen Substratoberfläche,
- Aufbringen von Referenz-Spots auf die ebene Substratoberfläche, sowie
- Aufbringen von Antigen-Spots auf die ebene Substratoberfläche,
wobei das Aufbringen der Antigen-Spots derart durchgeführt wird, dass die Referenz-Spots und die Antigen-Spots des Antigen-Chips gemäß einem erfindungsgemäßen Antigen-Chip nach einer der hier beschriebenen Ausführungsformen ausgeführt sind.

Das Herstellungsverfahren weist vorzugsweise ferner den Schritt eines Fragmentierens des Substrats auf, um mehrere Antigen-Chips zu erhalten, insbesondere an zwei Fragmentierungsstellen, deren Abstand zueinander größer ist als eine Breite des Substrates, innerhalb welcher die Gesamtheit aus Referenz-Muster und Antigenmuster vorhanden ist.

Vorzugsweise ist die Substratoberfläche eine Oberfläche eines Glas-Substrates oder eines mit einer Membran und/oder Folie beschichteten Glas-Substrates.

Das Aufbringen der Antigen-Spots kann mittels eines piezoelektrischen Mikrodosiergeräts durchgeführt werden, und die Substratoberfläche kann insbesondere eine Oberfläche eines Glas-Substrates oder eines mit einer Membran und/oder Folie beschichteten Glas-Substrates sein. Damit ist die Herstellung mit herkömmlichen Grundmaterialien und Herstellungsmaschinen durchführbar.

Vorgeschlagen wird ferner ein Kit zur Verwendung in einem Verfahren zum Detektieren von Antikörpern in einer flüssigen, biologischen Probe, welches einen erfindungsgemäßen Antigen-Chip nach einer der hier beschriebenen Ausführungsformen aufweist sowie ferner ein Konjugat, welches einen mit einem zweiten Farbstoff markierten sekundären Antikörper aufweist. Vorzugsweise ist der zweite Farbstoff ein Fluoreszenzfarbstoff, ein chromogenes Substrat, ein Enzym oder ein Substrat für eine Chemilumenszenzreaktion.

Der Chip kann als ein festes Substrat aufgefasst werden, etwa eine Glasplatte oder zum Beispiel Siliziumplatte. Der Chip kann auch aus Kunststoff oder auch aus Metall gefertigt sein. Der Chip kann lichtdurchlässig oder nicht lichtdurchlässig sein, um Durchlicht- oder Auflichtbeleuchtung bzw. -detektion zu unterstützen. Der Antigen-Chip kann in einem Scanner- oder Kamerasystem, insbesondere Fluoreszenzmikroskop, zur Untersuchung einer Probe eingesetzt werden und kann dann mit Beleuchtungslicht bzw. Anregungslicht bestrahlt werden.

Gemäß einer Ausführungsform der vorliegenden Erfindung ist die Substratoberfläche zur Fokussierung mikrostrukturiert. Die Mikrostrukturierung kann durch eine Oberflächenbehandlung (zum Beispiel Aufrauung) ausgeführt sein und kann eine Fokussierung auf die Substratoberfläche erleichtern. Verschiedene Verfahren zur Mikrostrukturierung, wie beispielsweise Spritzgießen, Heißprägen oder Imprint-Verfahren, sind im Stand der Technik bekannt. Es können verschiedene Mikrostruktur-Marker auf der Oberfläche aufgebracht sein, welche auch zur Identifizierung des Antigen-Chips, zur Identifizierung der Probe etc. verwendet werden können. Das Antigen-Substrat (der Chip) umfasst insbesondere eine Fläche mit einer Größe zwischen 1 mm x 1 mm und 2 mm x 4 mm.

Der Ausdruck "Chemilumineszenz", wie hierin verwendet, bezieht sich auf eine chemische Reaktion, bei der Energie spezifisch zu einem Molekül geleitet wird, was bewirkt, dass es elektronisch angeregt wird und anschließend ein Photon freisetzt, wodurch sichtbares Licht emittiert wird. Wärmeenergie wird für diese Reaktion nicht benötigt. Chemilumineszenz beinhaltet somit die direkte Umwandlung von chemischer Energie in Lichtenergie. Bevorzugt entsteht Chemilumineszenz durch die Reaktion eines Luminophors mit anderen Verbindungen. Diese Reaktionen können durch Enzyme katalysiert sein. In weiter bevorzugten Ausführungsformen der Erfindung ist der Luminophor aus der Gruppe bestehend aus Luminol und Derivate davon, Acridin und Derivate davon und Luciferine ausgewählt. Diese Verbindungen können durch verschiedene enzymatische Reaktionen zur Chemilumineszenz angeregt werden. Die entsprechenden Verbindungen und Reaktionen sind im Stand der Technik bekannt. In weiter bevorzugten Ausführungsformen der Erfindung ist das Luminophor Luminol oder ein Derivat davon, wobei durch eine von Peroxidase, insbesondere Meerettichperoxidase, katalysierte Reaktion Licht emittiert wird (Chemilumineszenz). In alternativen Ausführungsformen der Erfindung ist das Luminophor Acridin oder ein Derivat davon, insbesondere ein Acridiniumester oder Acridiniumsulfonamid, wobei durch eine von Phosphatase, insbesondere alkalische Phosphatase (AP), katalysierte Reaktion Licht emittiert wird (Chemilumineszenz). In weiteren alternativen Ausführungsformen der Erfindung ist das Luminophor ein Luciferin, insbesondere D-Luciferin, wobei durch eine von Luciferase katalysierte Reaktion Licht emittiert wird (Chemilumineszenz).

Ein "chromogenes Substrat", im Sinne der vorliegenden Erfindung, ist ein Reagenz, das zur Messung von Enzymaktivitäten eingesetzt werden kann. Das chromogene Substrat wird durch enzymatische Aktivität so verändert, dass ein direktes oder indirektes Produkt der enzymatischen Reaktion (photometrisch) quantifiziert werden kann. Das chromogene Substrat besteht z.B. aus einem Oligopeptid, an das ein Azofarbstoff, zum Beispiel Paranitroanilin, gekoppelt ist. Das Peptid imitiert die Schnittstelle, an der das zu untersuchende Enzym sein physiologisches Substrat spaltet. Durch die Enzymwirkung wird der Farbstoff freigesetzt. Die Farbentwicklung kann photometrisch quantifiziert und über eine Eichkurve die Enzymaktivität ermittelt werden. Das chromogene Substrat kann allerdings auch 5-Brom-4-chlor-3-indoxylphosphat (BCIP) und/oder Nitroblautetrazoliumchlorid (NBT) sein. Ein chromogenes Substrat kann z.B. kolorimetrisch nachgewiesen werden.

Die Antigene, welche in den verschiedenen Antigen-Spots enthalten sind, können ausgewählt und ausgebildet sein, Antikörper, die sich als Folge von Autoimmunerkrankungen, Allergien und Infektionskrankheiten bilden zu binden. Im vorliegenden Testsystem betrifft dies insbesondere antinukleäre und/oder extrahierbare antinukleäre Antikörper aus dem Bereich der Kollagenosen. Insbesondere können beispielsweise folgende Antigene (allein oder in beliebiger Kombination) in den Antigen-Spots enthalten sein: RNP/Sm, Sm, Scl-70, Rib.P0, Jo-1, SS-A, SS-B, dsDNA, Nukleosomen/Chromatin, Cenp-B, RNP A,C,68kDa, Ro-52, Ku, Histone, DFS70. Die diagnostizierbaren Kollagenosen können zumindest eine der folgende Formen enthalten: SLE (Systemic lupus erythematosus), PM, DM (Myositis), SS (Sjögren's Syndrom), CREST-Syndrom (limited cutaneous form of systemic sclerosis IcSSc), PSS (progressive systemic sclerosis), MCTD (mixed connective tissue disease, sharp syndrome), AID (autoimmune induced disease). Damit können verschiedene Krankheiten diagnostiziert werden.

Vorteilhafte Ausführungsformen der Erfindung sind Gegenstand der abhängigen Ansprüche und werden in der folgenden Beschreibung unter teilweise Bezugnahme auf die Fig.en näher erläutert.

Dabei zeigen:
Fig. 1 und 2 Antigen-Chips aus dem Stand der Technik,
Fig. 3a und 3b beispielhafte Ausführungsformen eines erfindungsgemäßen Antigen-Chips,
Fig. 4a und 4b eine zur Produktion eines erfindungsgemäßen Antigen-Chips mit Spots versehene Substratoberfläche sowie beispielhafte erfindungsgemäße Antigen-Chips,
Fig. 5a und 5b jeweilige Bilder eines beispielhaften Antigen-Chips in jeweiligen Farbkanälen aufgrund jeweiliger Färbung durch jeweilige Farbstoffe,
Fig. 6 ein beispielhaftes Spektrum eines Anregungslichtes sowie eines Fluoreszenzlichtes,
Fig. 7 ein weiteres Beispiel für ein Spektrum eines Anregungslichtes sowie jeweilige Fluoreszenzlichtspektren jeweiliger Fluoreszenzfarbstoffe,
Fig. 8 eine bevorzugte Ausführungsform einer erfindungsgemäßen Vorrichtung zum automatisierten Detektieren von Antikörpern in einer Probe,
Fig. 9 eine bevorzugte Ausführungsform einer erfindungsgemäßen Datennetzwerkvorrichtung,
Fig. 10 bevorzugte Schritte zur Durchführung des erfindungsgemäßen Verfahrens zum automatisierten Detektieren von Antikörpern einer flüssigen, biologischen Probe gemäß einer bevorzugten Ausführungsform,
Fig. 11a bevorzugte Schritte im Zuge einer Detektion eines zweiten Referenz-Musters,
Fig. 11b bevorzugte Schritte im Zuge eines Erstellens einer Zuordnungs-Information,
Fig. 11c eine beispielhafte Ausführungsform eines Abfolge-Datensatzes,
Fig. 12 eine Ausführungsform eines Datensatzes zur Indizierung von Bindungsmaßen,
Fig. 13 bevorzugte Schritte zur Durchführung im Zuge eines Verfahrens zur Herstellung eines Antigen-Chips,
Fig. 14 bevorzugte Schritte zur Durchführung zur Detektion eines Referenz-Musters sowie jeweiliger Antigen-Spot-Muster,
Fig. 15 bevorzugte Schritte zur Durchführung im Zuge eines Erkennens mehrerer potentieller Muster auf Basis einer ersten Bildinformation sowie eines Detektierens jeweiliger Spotabstände von Spots von Mustern,
Fig. 16 eine Verwendung von Positions-Informationen im Zuge eines Bestimmens von Bindungsmaßen auf Basis einer zweiten Bildinformation,
Fig. 17 bevorzugte Schritte zur Durchführung im Zuge des Verfahrens zum automatisierten Detektieren von Antikörpern oder des Verfahrens für eine automatisierte Bildverarbeitung zum Detektieren von Antikörpern,
Fig. 18 eine bevorzugte Ausführungsform eines erfindungsgemäßen Objektträgers mit einer Mehrzahl an Antigen-Chips,
Fig. 19 eine detaillierte Darstellung eines Feldes mit mehreren Antigen-Chips,
Fig. 20a eine erste Bildinformation bzw. ein erstes Bild, welches eine Färbung von Referenz-Spots und Antigen-Spots in einem ersten Farbkanal repräsentiert,
Fig. 20b die erste Bildinformation bzw. das erste ein Bild, wie in Fig. 20a gezeigt, zusammen mit indizierten Positions-Informationen von Spots,
Fig. 21a eine zweite Bildinformation bzw. ein zweites Bild, welches eine Färbung von Referenz-Spots und Antigen-Spots in einem zweiten Farbkanal indiziert,
Fig. 21b die zweite Bildinformation bzw. das zweite Bild, wie in Fig. 21a gezeigt, zusammen mit indizierten Positionen von Spots, wie aus dem ersten Farbkanal bzw. der ersten Bildinformation gewonnen,
Fig. 22a eine beispielhafte Ausführungsform eines Antigen-Chips, bei welchem das Referenz-Muster einen anderen Nachbar-Abstand zu einem nächsten Nachbar-Muster aufweist als die Antigen-Spot-Muster,
Fig. 22b eine beispielhafte Ausführungsform eines Antigen-Chips, bei welchem das Referenz-Muster Referenz-Spots mit einer solchen gleichen Größe aufweist, welche sich von den Größen der Antigen-Spots der Antigen-Spot-Muster unterscheidet.

Wie bereits zuvor ausgeführt, es ist aus dem Stand der Technik bekannt, Antigen-Chips mit Spots unterschiedlicher Antigen-Typen in einer Art Schachbrettmuster anzuordnen. Wie zuvor erläutert, kann es hierbei im Zuge einer Herstellung eines Antigen-Chips vorkommen, dass ein Antigen-Chip AC, wie in der Fig. 2 gezeigt, eine Ausrichtung von Antigen-Spots AS aufweist, welche nicht zu einem später anzuwendenden Raster passt, welches verwendet wird, um die Spots AS aufgrund ihrer genauen Position entsprechenden Antigen-Typen zuzuweisen, so dass eine Detektion einer Bindung von Antikörpern einer flüssigen Probe an bestimmte Antigen-Typen durch Auswertung in einem sogenannten zweiten Farbkanal erfolgen kann.

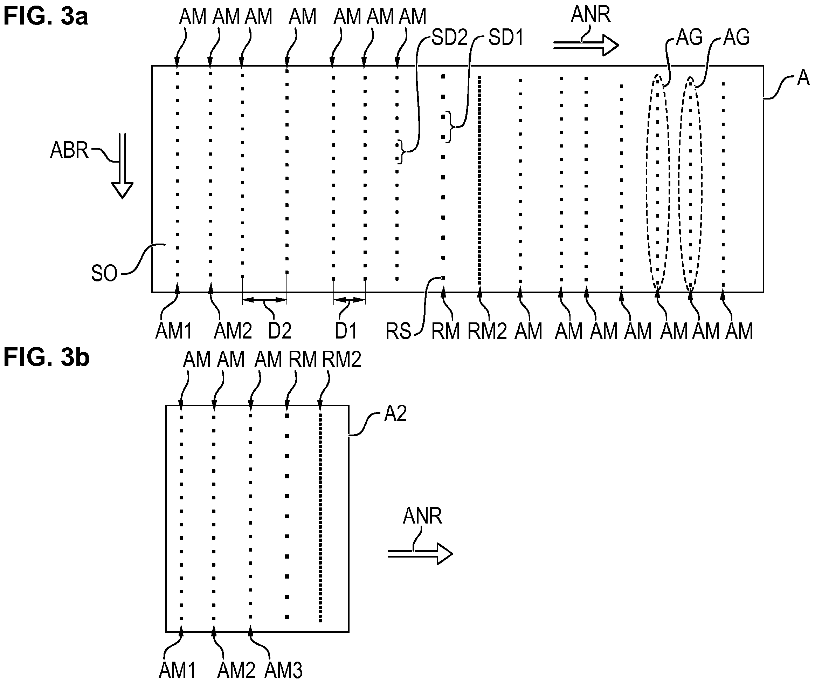

Die Fig. 3a zeigt eine beispielhafte Ausführungsform eines Antigen-Chips A, bei welchem auf einer Substratoberfläche SO Antigen-Spots AS in Antigen-Spot-Mustern AM beabstandet zueinander aufgebracht sind, wobei die Antigen-Spots AS einen gleichen, gemeinsamen Farbstoff bzw. ersten Farbstoff aufweisen.

Die Antigen-Spots bilden jeweilige Antigen-Spotmengen AG aus, welche wiederum jeweilige regelmäßige Antigen-Spot-Muster AM ausbilden. In diesem Ausführungsbeispiel sin dies Muster AM, bei welchen Antigen-Spots entlang einer Linie von oben nach unten verlaufend angeordnet sind.

Der Antigen-Chip A weist ferner ein Referenz-Muster RM mit Referenz-Spots auf, welche einen Spotabstand SD1 zueinander aufweisen.

Der Spotabstand SD2 der Antigen-Spots der Antigen-Spot-Muster AM ist unterschiedlich zu dem Spotabstand SD1 des Referenz-Musters RM.

Der Regelmäßigkeitsparameter der Muster RM, AM ist hierbei der jeweilige Spotabstand SD1, SD2 innerhalb der jeweiligen Muster RM bzw. AM. Das Referenz-Muster RM unterscheidet sich also gegenüber den Antigen-Spot-Mustern AM hinsichtlich des Wertes dieses Regelmäßigkeitsparameters bzw. hinsichtlich seiner Regelmäßigkeit. Die Muster RM, AM sind entlang einer Anordnungsrichtung ANR angeordnet. Die Muster RM, AM weisen jeweils eine Ausbreitungsrichtung ABR auf, welche senkrecht zur Anordnungsrichtung ANR der Muster AM,RM ist.

Die Spots der Muster RM, AM weisen einen gleichen, gemeinsamen Farbstoff auf.

Es sei angenommen, dass die Antigen-Spots des Antigen-Spot-Musters AM1 ein gleiches, gemeinsames Antigen bzw. einen gleichen, gemeinsamen Antigentyp aufweisen, welcher sich von einem Antigentyp bzw. einem Antigen des Musters AM2 unterscheiden.

Wird ein solcher Antigen-Chip A mit all seinen Spots der Muster RM, AM mit einer biologischen Probe wie beispielsweise einem Blutserum inkubiert, so kann es für die Spots der unterschiedlichen Antigen-Spot-Muster AM1, AM2 aufgrund der unterschiedlichen Antigen-Typen der Muster AM1, AM2 zu unterschiedlich starken Bindungen von Antikörpern aus der biologischen, flüssigen Probe an die jeweiligen Antigen-Typen kommen.

Eine solche Bindung ist ein sogenannter erster Antikörper aus der biologischen, flüssigen Probe soll dann später detektiert werden.

Vorzugsweise weist der Antigen-Chips A ein weiteres, zweites Referenz-Muster RM2 auf, welches sich mit seinen Referenz-Spots hinsichtlich seiner Regelmäßigkeit bzw. seinem Wert des Regelmäßigkeitsparameters sowohl von dem ersten Referenz-Muster RM als auch den Antigen-Spot-Muster AM unterscheidet. Dies ist der Fall, da das Referenz-Muster RM2 einen Spotabstand SD3 aufweist, welcher sich klar von den Spotabständen SD1, SD2 unterscheidet.

Der gemeinsame, gleiche Farbstoff der Spots der Muster RM, RM2, AM ist vorzugsweise in einem ersten Farbkanal als Rotkanal sichtbar.

Die Regelmäßigkeit des Referenz-Musters RM unterscheidet sich von den Antigen-Spot-Mustern AM und insbesondere auch dem zweiten Referenz-Muster RM2 durch eine Variation eines gemeinsamen Regelmäßigkeitsparameters der Muster RM, AM und insbesondere RM2. Im Sinne dieser Anmeldung kann eine Regelmäßigkeit auch als eine Periodizität eines Musters bezeichnet werden, wobei der Regelmäßigkeitsparameter ein Periodizitätsparameter ist. Sich unterscheidende Spot-Muster weisen dann unterschiedliche Werte hinsichtlich eines gleichen Regelmäßigkeitsparameters der Spot-Muster auf.

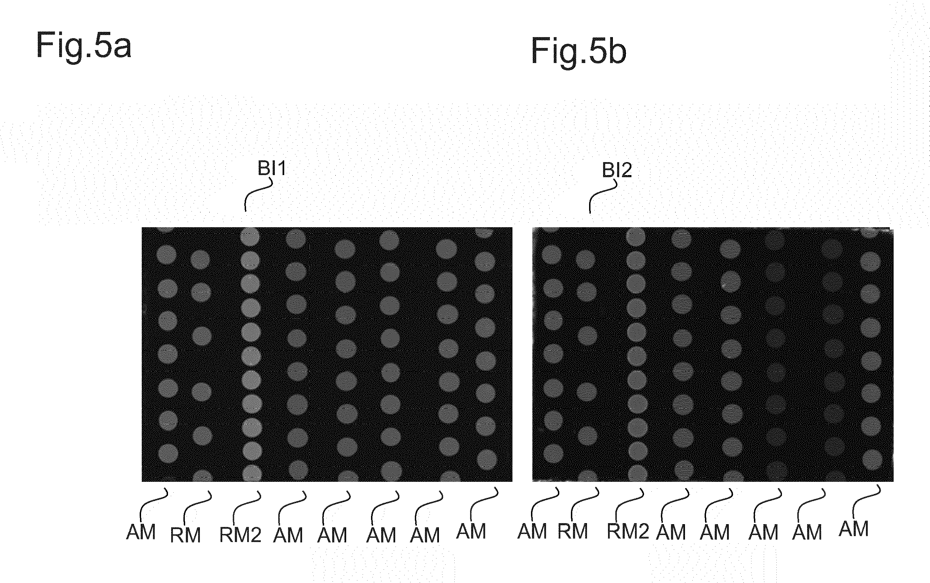

Die Fig. 5a und 5b zeigen eine erste Bildinformation BI2 bzw. eine zweite Bildinformation BI2 mit jeweiligen Färbungen in unterschiedlichen Farbkanälen durch jeweilige Farbstoffe.

Die erste Bildinformation BI1 zeigt ein beispielhaftes Referenz-Muster RM sowie ein beispielhaftes und vorzugsweise vorhandenes zweites Referenz-Muster RM2 in dem ersten Farbkanal aufgrund einer Färbung des ersten Farbstoffes. Ferner sind Antigen-Spot-Muster AM in der ersten Bildinformation BI1 sichtbar aufgrund der Färbung durch den ersten Farbstoff in dem ersten Farbkanal.

Durch Vorsehen bzw. Verwendung eines vorgeschlagenen zweiten Referenz-Musters RM2 wird es ermöglicht, dass im Zuge einer automatisierten Bildauswertung eine anzunehmende Abfolge von Antigen-Typen in der Anordnungsrichtung ANR bezogen auf die einzelnen Antigen-Spot-Muster AM dann korrigiert werden kann, wenn der in Fig. 3a gezeigte Antigen-Chip A um 180° rotiert ist. Durch Lage des ersten Referenz-Musters RM und des zweiten Referenz-Musters RM2 zueinander lässt sich dann eine solche unerwünschte Rotation des Antigen-Chips A in einer Bildinformation des ersten Farbkanals detektieren und so die Lage bzw. die Position der Antigen-Spot-Muster entlang der angenommenen Anordnungsrichtung ANR auch in dem Fall einer unerwünschten 180° Rotation des Antigen-Chips korrigieren.

Der erste Farbstoff ist vorzugsweise ein Fluoreszenzfarbstoff. Alternativ ist der erste Farbstoff ein chromogenes Substrat, welches zum Zwecke einer chromogenen Färbung eine Bindung mit einem Enzym eingehen kann, welches durch ein weiteres Konjugat im Zuge einer weiteren Inkubation eines Antigen-Chips beigefügt werden kann. Die Verfärbung aufgrund einer Bindung des chromogenen Substrats mit einem Enzym kann als ein Verfärbungsprodukt angesehen werden, welches photometrisch erfassbar ist.

Die zweite Bildinformation BI2 aus der Fig. 5b zeigt, dass nicht für alle Antigen-Spot-Muster AM eine Färbung im zweiten Farbkanal aufgrund des zweiten Farbstoffes signifikant vorhanden ist. Dies ist vermutlich der Fall, da an die entsprechenden Antigen-Typen der entsprechenden Antigen-Spot-Muster keine wesentliche bzw. signifikante Bindung von Antikörpern der flüssigen, biologischen Probe erfolgte, so dass bei einer Inkubation des Antigen-Chips mit einem zweiten Konjugat, welches einen zweiten Antikörper mit einer Markierung durch den zweiten Farbstoff enthält, dann die zweiten Antikörper nicht an erste Antikörper aus der flüssigen biologischen Probe gebunden wurden.

Der zweite Farbstoff ist vorzugsweise ein Fluoreszenzfarbstoff.

Vorzugsweise ist der zweite Fluoreszenzfarbstoff ein chromogenes Substrat, welches an ein Enzym eine Bindung eingehen kann, wobei das Enzym in einem Konjugat enthalten sein kann, welches zum Inkubieren der Spots in einem weiteren Prozessierungsschritt verwendet werden kann. Das Enzym geht dann mit dem chromogenen Substrat ein Verfärbungsprodukt ein, welches photometrisch erfassbar ist.

Vorzugsweise ist der zweite Farbstoff ein Enzym, so dass bei einer Inkubation der Spots mit einem Konjugat, welches ein chromogenes Substrat aufweist, das Enzym mit dem chromogenen Substrat eine Bindung eingeht um ein Verfärbungsprodukt zu erzeugen, welches photometrisch erfassbar ist.

Vorzugsweise ist der zweite Farbstoff ein Enzym, welches im Zuge eines weiteren Prozessierungsschrittes durch Inkubation der Spots mit einem Konjugat, welches ein weiteres Substrat aufweist, eine Bindung bzw. Interaktion eingehen kann, so dass Strahlung aufgrund einer Chemilumineszenzreaktion emittiert wird.

Vorzugsweise ist der zweite Farbstoff ein Substrat für eine Chemilumineszenzreaktion, so dass bei Inkubieren der Spots mit einem Konjugat, welches ein Enzym aufweist, das wiederum eine Reaktion mit dem Substrat eingehen kann, die Chemilumineszenzreaktion hervorrufen wird, so dass Chemilumineszenzstrahlung emittiert wird.

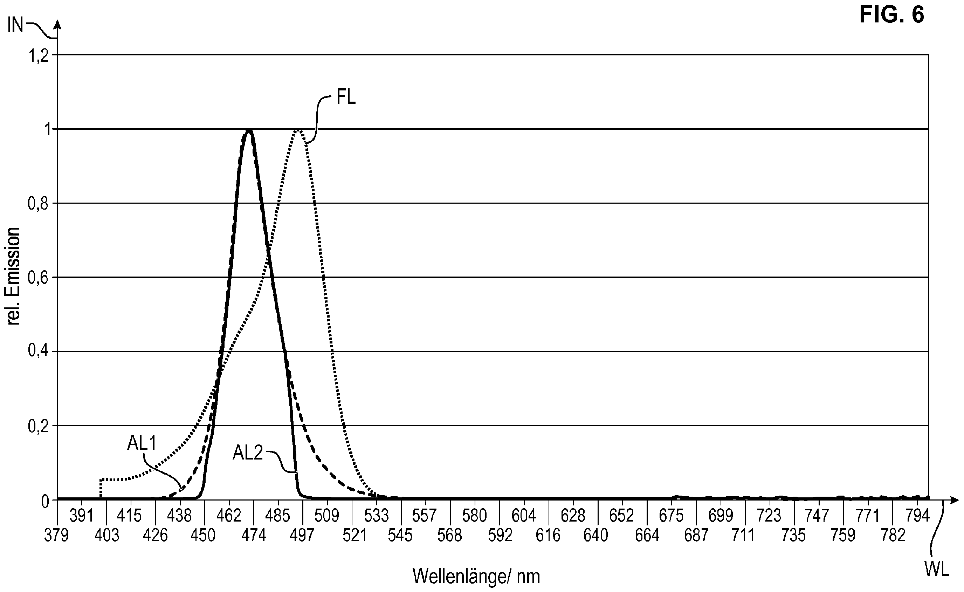

In dem Fall, dass ein Farbstoff ein Fluoreszenzfarbstoff ist, kann beispielsweise eine Fluoreszenzstrahlung FL durch ein Anregungslicht AL1 oder AL2, wie in der Fig. 6 gezeigt, angeregt werden. Das Fluoreszenzlicht FL ist vorzugsweise das Licht, welches von dem ersten Fluoreszenzfarbstoff emittiert wird. Die Achsen der in Darstellung der Fig. 6 zeigen auf der Abszisse die Wellenlänge WL sowie auf der Ordinate die Intensität IN.

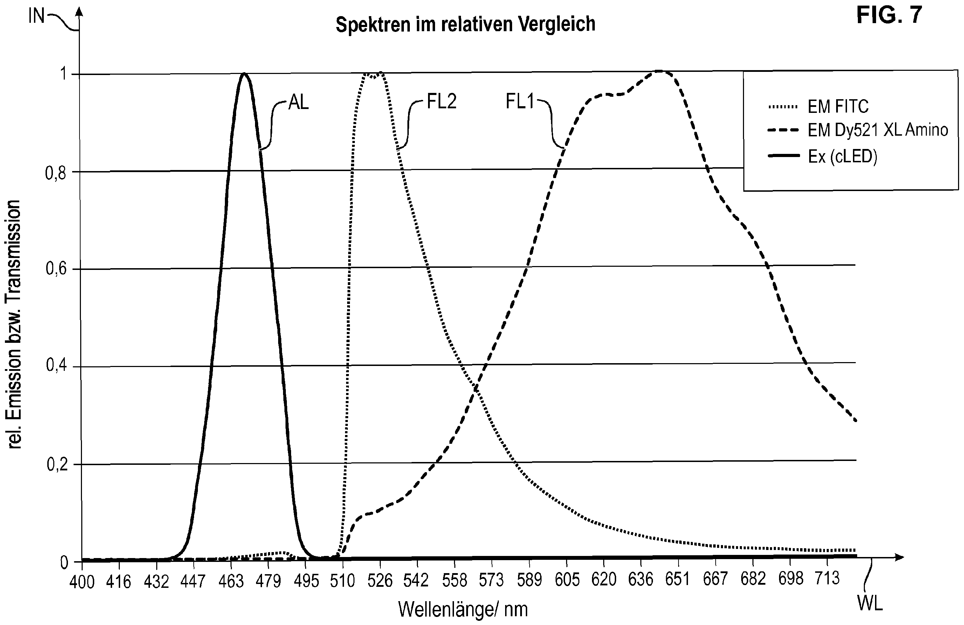

Die Achsen der in Darstellung der Fig. 7 zeigen auf der Abszisse die Wellenlänge WL sowie auf der Ordinate die Intensität IN. Die Fig. 7 zeigt in einem Diagramm mit einer Wellenlänge WL auf der Abszisse sowie einer Intensität IN auf der Ordinate ein Spektrum eines Anregungslichtes AL, ein Spektrum FL1 einer ersten Fluoreszenzstrahlung durch einen ersten Fluoreszenzfarbstoff sowie ein Spektrum FL2 einer zweiten Fluoreszenzstrahlung durch einen zweiten Fluoreszenzfarbstoff.

Die erste Fluoreszenz FL1 kann Aussendung von Licht in einem ersten Wellenlängenbereich umfassen und die zweite Fluoreszenz FL2 kann Aussendung von Licht in einem zweiten Wellenlängenbereich umfassen, wobei der erste Wellenlängenbereich und der zweite Wellenlängenbereich nur einen geringen oder im Wesentlichen keinen Überlappungsbereich aufweisen, etwa einen Überlappungsbereich oder eine Überlappung, welcher zum Beispiel weniger als 10%, insbesondere weniger als 5%, eines Integrals einer Emission der ersten Fluoreszenz über die Wellenlänge entspricht.

Der erste Fluoreszenzfarbstoff kann zum Beispiel in einem roten Wellenlängenbereich fluoreszieren, zum Beispiel in einem Bereich zwischen 550 nm und etwa 800 nm mit einem Maximum zwischen 600 nm und 660 nm. Dazu kann beispielsweise der erste Fluoreszenzfarbstoff der Farbstoff DY521XL sein. Die Anregung des ersten Fluoreszenzfarbstoffes kann in einem Frequenzbereich zum Beispiel zwischen 450 nm und 500 nm mit einem Maximum zum Beispiel zwischen 550 nm und 580 nm erfolgen. Die Anregung des zweiten Fluoreszenzfarbstoffes kann im Wesentlichen in demselben Wellenlängenbereich wie die Anregung des ersten Fluoreszenzfarbstoffes erfolgen. Der zweite Fluoreszenzfarbstoff kann zum Beispiel in einem grünen Wellenlängenbereich fluoreszieren, zum Beispiel in einem Bereich zwischen 500 nm und etwa 600 nm mit einem Maximum zwischen 510 nm und 530 nm. Dazu kann beispielsweise der erste Fluoreszenzfarbstoff der Farbstoff FITC sein.

Damit ist in einer Vorrichtung zum automatisierten Detektieren von Antikörpern in einer Probe beispielsweise nur eine einzige Beleuchtungslichtquelle zur Erzeugung des Anregungslichts erforderlich, welche relativ schmalbandig ausgeführt sein kann. Damit kann vorteilhaft das Anregungslicht herausgefiltert werden, bevor die erste Fluoreszenz durch eine erste Kamera und die zweite Fluoreszenz durch eine zweite Kamera oder beide Fluoreszenzsignale durch die erste oder die zweite Kamera (z.B. nach einem Filterwechsel) detektiert werden, ohne im Wesentlichen störendes Anregungslicht zu detektieren.

Zurückkommend zur Fig. 5a kann angemerkt werden, dass vorzugsweise die Referenz-Spots der Referenz-Spot-Menge RM den Farbstoff in einer Farbstoffkonzentration aufweisen können, welche größer ist als die Farbstoffkonzentrationen, in welchen die Antigen-Spots bzw. die Antigen-Spotmengen bzw. Antigen-Spot-Muster AM und insbesondere auch das Referenz-Muster RM2 den ersten Farbstoff bzw. den gleichen, gemeinsamen Farbstoff aufweisen. Vorzugsweise kann auch das zweite Referenz-Muster RM2 diesen gleichen, gemeinsamen Farbstoff in einer Farbstoffkonzentration aufweisen, welche größer ist als die Farbstoffkonzentrationen, in welchen die Antigen-Spots der Antigen-Spotmengen AM den gleichen, gemeinsamen Farbstoff aufweisen.

Würden die Spots der Referenz-Spot-Menge RM - und insbesondere auch der zweiten Referenz-Spot-Menge RM2 - den gleichen, gemeinsamen Farbstoff in einer höheren Farbstoffkonzentration aufweisen, so könnte hierdurch eine Detektion des Referenz-Musters RM - und insbesondere auch des weiteren Referenz-Musters RM2 - im Zuge einer automatisierten Bildverarbeitung erleichtert werden, da die Farbintensitäten dieser Spots in einer Bildinformation größer wären als die der Antigen-Spots der Antigen-Spot-Mengen.

Zurückkommend zur Fig. 3a kann ferner angemerkt werden, dass das Referenz-Muster RM ein Referenzlinienmuster ausbildet und dass ferner die Antigen-Spot-Muster AM ebenfalls Referenzlinienmuster ausbilden, wobei die Linien der Muster RM und AM eine gleiche Ausbreitungsrichtung aufweisen. Ebenfalls bildet das Referenz-Muster RM2 ein Referenzlinienmuster aus. Dies ist auch als weiteres oder zweites Referenzlinienmuster zu bezeichnen.

Innerhalb der Linienmuster RM, RM2, AM folgen die jeweils zugehörigen Spots dieser Muster im Wesentlichen in jeweils regelmäßigen, vorzugsweise im Wesentlichen äquidistanten Abständen aufeinander, wobei der regelmäßige, vorzugsweise im Wesentlichen äquidistante Abstand der Referenz-Spots des Referenzlinienmusters RM sich von den regelmäßigen, vorzugsweise im Wesentlichen äquidistanten Abständen der Antigen-Spots der Antigenlinienmuster AM unterscheidet. Ist neben dem Referenz-Muster RM ein weiteres, zweites Referenz-Muster RM2 als ein Linienmuster vorhanden, so unterscheidet sich dieses Muster RM2 sowohl von dem ersten Referenz-Muster RM als auch den Antigenlinienmustern AM darin, dass die zugehörigen Spots des Musters RM2 sich durch ihren regelmäßigen, vorzugsweise im Wesentlichen äquidistanten Abstand von den regelmäßigen, vorzugsweise im Wesentlichen äquidistanten Abständen der Antigen-Spots der Antigenlinienmuster AM als auch der Spots des ersten Referenz-Musters RM2 unterscheiden.

Die Fig. 3b zeigt eine bevorzugte Variante A2 eines erfindungsgemäßen Antige-Chips A2, bei welchem nur drei Antigen-Spot-Muster AM1, AM2, AM3 auf dem Antigen-Chip A2 vorhanden sind. Hierbei handelt es sich um eine Untermenge von Mustern, welche bereits in dieser Anordnung auf dem Antigen-Chip A aus der Fig. 3a vorhanden sind. Auch hierbei sind die Muster RM, RM2, AM in einer Anordnungsrichtung ANR angeordnet. Zur Verdeutlichung eines der Vorteile des erfindungsgemäßen Verfahrens und des erfindungsgemäßen Antigen-Chips sei angenommen, dass es angestrebt sei, einen Antigen-Chip, wie in der Fig. 3b gezeigt, herzustellen.

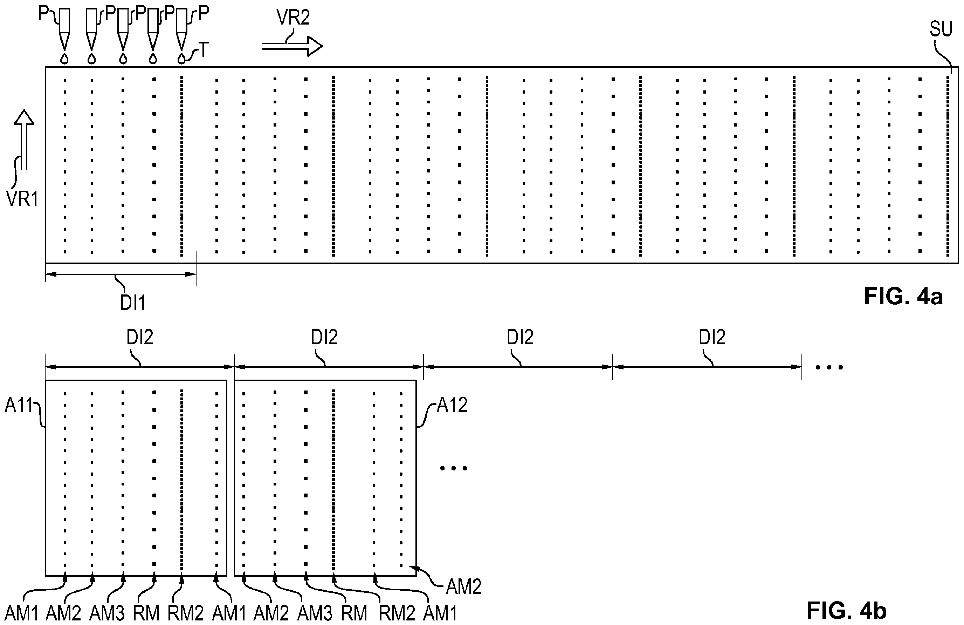

Die Fig. 4a zeigt ein Substrat bzw. eine Substratoberfläche SU. Die Substratoberfläche SU ist vorzugsweise eine Oberfläche eines Glassubstrates oder eines mit einer Membran und/oder Folie beschichteten Glassubstrates. Auf dem Substrat SU sollen unterschiedliche Muster RM, RM2, AM entlang einer Anordnungsrichtung ANR auf das Substrat aufgebracht werden. Hierzu werden die Spots der jeweiligen Muster RM, RM2, AM mittels jeweiliger Pipetten P auf das Substrat SU aufgebracht. Die Pipetten P können hierbei ortsfest sein, so dass durch jeweilige Tropfen T die Pipetten P jeweilige Spots für eine jeweilige Spot-Menge vorzugsweise als ein Linienmuster auf die Substratoberfläche SU aufbringen, wobei in einer Vorschub-Richtung VR1 das Substrat SU so gegenüber den Pipetten P verschoben werden kann, dass sich die zuvor erwähnten regelmäßigen Muster ausbilden. Es bilden also die Referenz-Spots der Referenz-Spot-Menge RM ein Referenzlinienmuster aus und die Antigen-Spots der jeweiligen Antigen-Spotmengen AM jeweilige Antigenlinienmuster. Gleiches gilt für das vorzugsweise vorhandene zweite Referenz-Muster RM2.

Wurde die Substratoberfläche SU in der Vorschub-Richtung VR1 soweit gegenüber den Pipetten P verschoben, dass sich die entsprechenden Linienmuster RM, RM2, AM ausgebildet haben, so ergibt sich eine Distanz DI1, welche in der Anordnungsrichtung der Muster ANR die Mindestbreite des herzustellenden Antigen-Chips vorgibt. Wird der Substratträger SU dann entgegen der Vorschub-Richtung VR1 in die ursprüngliche, in der Fig.4a dargestellte Position zurückverfahren, so kann dann durch ein Versetzen bzw. Vorschieben des Substratträgers SU in der Vorschub-Richtung VR2 um die Distanz DI1 wiederum ein neuer Satz an entsprechenden Referenz-Mustern RM, RM2 und Antigen-Spot-Mustern AM ausgebildet werden, wobei dann eben wiederum auch der Substratträger SU in der Vorschub-Richtung VR1 vorgeschoben wird, um eine Relativbewegung des Substratträgers SU gegenüber den Pipetten P zu bewirken. Um die entsprechenden Linienmuster RM, RM2, AM mit unterschiedlichen regelmäßigen Abständen ihrer jeweiligen Spots auszubilden, müssen also die entsprechenden Pipetten P nur in der Weise angesteuert werden, dass die Tropfen T mit unterschiedlichen Frequenzen bzw. unterschiedlichen Abständen entlang der Vorschub-Richtung VR1 ausgebildet werden. Ein Ausbilden der Muster RM, RM2, AM als Linienmuster erlaubt eine besonders effektive Herstellung des erfindungsgemäßen Antigen-Chips. Es können auf dem Substratträger SU also dann Muster für mehrere Antigen-Chips in serieller Abarbeitung erzeugt werden und es muss nicht für jeden Antigen-Chip ein eigener Substratsträger SU verwendet werden. Das Aufbringen durch die Pipettenköpfe erfolgt insbesondere durch einen sogenannten Piezo-Druck mittels eines piezoelektrischen Mikrodosiergerätes als eine besondere Ausbildungsform der Pipetten P. Es kann also festgehalten werden, dass ein Aufbringen von Referenz-Spots und Antigen-Spots auf die ebene Substratoberfläche SU in der Weise ausgeführt wird, dass sich ein Antigen-Chip gemäß der zuvor beschriebenen Ausführungsform ergibt.

Die Fig. 4b verdeutlicht nun einen besonderen Vorteil des erfindungsgemäßen Antigen-Chips. Die Substratoberfläche aus der Fig. 4a könnte exakt in der Weise fragmentiert werden, dass immer exakt nach der Distanz DI1 das Substrat SU parallel zu der Ausbreitungsrichtung der Muster, also senkrecht zur Anordnungsrichtung ANR der Muster, fragmentiert wird. Es würde sich dann eine Lage des Referenz-Musters RM und der Antigen-Spot-Muster AM auf den verschiedenen, derart herzustellenden Antigen-Chips an immer gleicher, sich wiederholender Stelle bzw. Position auf den entsprechenden Antigen-Chip ergeben. Aufgrund der erfindungsgemäßen Ausbildung der Antigen-Spot-Muster AM und des Referenz-Musters RM als auch insbesondere des zweiten Referenz-Musters RM2 wird es jedoch, wie in der Fig. 4b illustriert, möglich, ein solches Fragmentieren an wenigstens einer, insbesondere an zwei Stellen, des Substrats SU an einer nur ungefähr zu wählenden Distanz DI2 vorzunehmen, so dass sich entsprechende Antigen-Chips A11 sowie A12 aus der Fig. 4b ergeben können. Wesentlich ist, dass die Distanz DI2, nach welcher eine Fragmentierung des Substratträgers SU erfolgt, mindestens genauso groß ist wie die Distanz DI1 oder aber größer als diese Distanz DI1. Die zu wählende Distanz DI2 muss ferner lediglich kleiner sein als das Doppelte der Distanz DI1, um eine vollständige Wiederholung der Gesamtheit aller Muster AM, RM, RM2 zu vermeiden. Zwar ergibt sich dann auf dem resultierenden Antigen-Chip A11 im rechten Bereich eine Wiederholung des Antigen-Spot-Musters AM1, jedoch kann die Abfolge der entsprechenden Antigen-Typen der Antigen-Spot-Muster AM1, AM2, AM3 aufgrund der Unterscheidbarkeit dieser Muster gegenüber dem Referenz-Mustern RM und insbesondere dem Referenz-Muster RM2 im Zuge einer automatisierten Bildauswertung verwendet werden, um für wenigstens jeden Antigentyp bzw. jedes der Antigen-Spot-Muster AM1, AM2, AM3 eine Bindung von Antikörpern zu detektieren. Der darauffolgende hergestellte Antigen-Chip A12 weist durch Wahl der Fragmentierungsdistanz DI2 gegenüber der Wiederholungsdistanz DI1 der Antigen-Spot-Muster und des Referenz-Musters als auch insbesondere des zweiten Referenz-Musters dann wenigstens jeden Typ eines Antigen-Spot-Musters AM1, AM2, AM3 als auch das Referenz-Muster RM und insbesondere auch das zweite Referenz-Muster RM2 wenigstens einmal auf. Erst durch die erfindungsgemäße Ausbildung der Antigen-Spot-Muster und auch des wenigstens einen Referenz-Musters RM ergibt sich die Möglichkeit, das Fragmentieren des Substrates SU nicht an festen Positionen, wie durch die Distanz DI1 vorgegeben, vornehmen zu müssen, sondern diese Distanz von Fragmentierungsstellen deutliche freier bzw. ungenauer wählen zu können. In der Lösung nach dem Stand der Technik ist es notwendig, wie zuvor unter Bezug auf die Fig. 2 und 1 erläutert, dass ein jeweiliger Antigen-Spot ASX auf einem entsprechenden Substrat bzw. innerhalb eines ansprechenden Musters an einer ganz genauen, entsprechenden Position vorkommt, um eine Zuordnung des Spots ASX zu einem bestimmten Antigen-Typen korrekt vornehmen zu können. Diese Anforderung wird durch den erfindungsgemäßen Antigen-Chip sowie das vorgeschlagene Verfahren zum automatisierten Detektieren von Antikörpern als auch das vorgeschlagene Verfahren für eine automatisierte Bildverarbeitung besonders geschickt umgangen und führt zu einer besonders einfachen Herstellungsweise von Antigen-Chips A11, A12.

Die Referenz-Spots RM des Referenz-Musters können ferner ebenfalls noch vorzugsweise ein Antigen aufweisen, so dass dann eine Bindung von Antikörpern der biologischen Probe, an welche wiederum zweite Antikörper eines Konjugats unter Markierung mit einem zweiten Farbstoff anbinden können, detektiert werden kann und somit ein weiterer, zusätzlicher Antigentyp hinsichtlich einer Detektion von Antikörpern in der biologischen Probe verwendet werden kann.

Vorzugsweise weisen die Referenz-Spots des Referenz-Musters RM als auch insbesondere des Referenz-Musters RM2 Antikörper auf, insbesondere in Form von anti-human IgG. Ein solcher Antikörper kann dann eine Bindung mit einem Antikörper einer flüssigen Patientenprobe, beispielsweise einem Blutserum, eingehen, so dass nach weiterer Inkubation der Referenz-Spots mit einem Konjugat, welches wiederum einen mit einem zweiten Farbstoff markierten zweiten Antikörper aufweisen kann, eine Bindung des zweiten Farbstoffs an die Referenz-Spots gegeben ist. Hierdurch kann dann später im Zuge einer automatisierten Bildverarbeitung als auch eines Verfahrens zum Detektieren von Antikörpern kontrolliert werden, ob der Antigen-Chip in korrekter Weise mit der Probe und/oder dem Konjugat inkubiert wurde. Die Referenz-Spots RM, RM2 können also als eine Inkubationskontrolle verwendet werden.

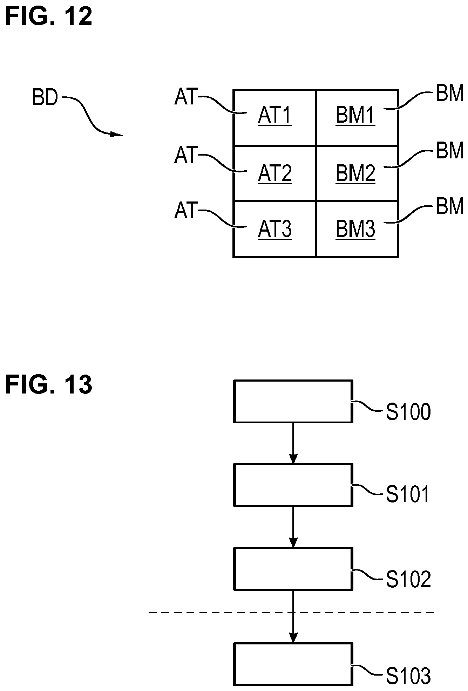

Fig. 13 zeigt noch einmal Schritte des vorgeschlagenen Herstellungsverfahrens. In dem Schritt S100 erfolgt ein Bereitstellen eines Substrates mit einer ebenen Substratoberfläche. In dem Schritt S101 erfolgt ein Aufbringen von Referenz-Spots auf die ebene Substratoberfläche. In einem Schritt S102 erfolgt ein Aufbringen von Antigen-Spots auf die ebene Substratoberfläche. Hierbei erfolgt das Aufbringen der Antigen-Spots und der Referenz-Spots zur Erlangung eines Antigen-Chips gemäß einer Ausführungsform in der zuvor beschriebenen Art und Weise. In einem vorzugsweise vorzunehmenden Schritt S103 erfolgt ein Fragmentieren des Substrats, um mehrere Antigen-Chips zu erhalten. Das Fragmentieren erfolgt unter Einhaltung einer Distanz zweier Fragmentierungsstellen, welche bei einer repetitiven Abfolge von Antigen-Spot-Mustern und Referenz-Spot-Mustern mindestens so groß ist wie eine Distanz, welche diese repetitive Abfolge in einer Anordnungsrichtung der Muster beschreibt.

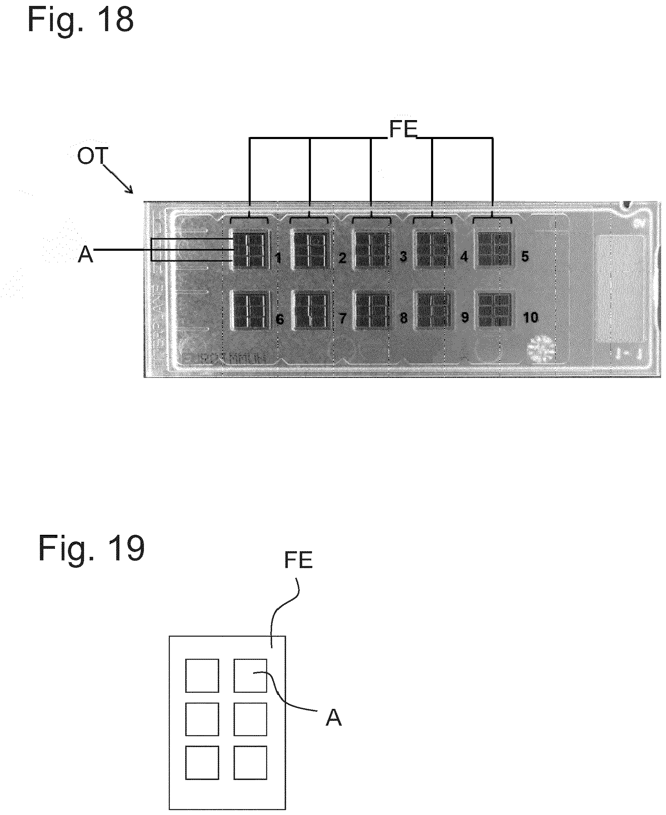

Wurden mehrere Antigen-Chips nach der zuvor beschriebenen Art und Weise hergestellt, so können mehrere Antigen-Chips zusammengefasst auf einem Objektträger bereitgestellt werden. Hierzu zeigt die Fig.18 einen Objektträger OT, welche auf unterschiedlichen Feldern FE jeweils mehrere Antigen-Chips A aufweist.

Fig. 19 zeigt hierzu eine detaillierte Darstellung eines einzelnen Feldes FE mit mehreren Antigen-Chips A.

Wie in den Fig. 3a, 3b, 4a sowie 4b dargestellt, können sich die Antigen-Spot-Muster AM sowie das Referenz-Muster RM dadurch unterscheiden, dass sie eine jeweilige unterschiedliche Periodizität bzw. einen unterschiedlichen regelmäßigen Abstand von Antigen-Spots zueinander aufweisen. Diese Ausbildung der Antigen-Spot-Muster AM und des Referenz-Musters RM sei nur beispielhaft hier in dieser Weise illustriert. Vorzugsweise besteht die Unterscheidung des Referenz-Musters RM, insbesondere auch des zweiten Referenz-Musters RM2, gegenüber den Antigen-Spot-Mustern AM darin, dass das Referenz-Muster RM entlang der Anordnungsrichtung ANR zu einem nächsten unmittelbar benachbarten Muster einen solchen unmittelbaren Nachbar-Abstand aufweist, welcher sich von den anderen Nachbarabständen der Antigen-Spot-Muster AM unterscheidet. Hierbei müssen also die Muster RM, AM nicht unterschiedliche regelmäßige Abstände ihrer jeweiligen Spots innerhalb der Muster RM, AM aufweisen, sondern können sich aufgrund einer Distanz bzw. eines Nachbar-Abstandes hin zum nächsten Muster in bzw. entlang der Anordnungsrichtung ANR unterscheiden.

Ferner ist es möglich, dass sich das Referenz-Muster RM, und insbesondere auch das zweite Referenz-Muster RM2, derartige Spots aufweist, welche eine gleiche Größe haben, wobei sich die Größe der Spots des Referenz-Musters RM von den Größen der Antigen-Spots der Antigen-Spot-Muster AM unterscheidet. Hierbei können also das Referenz-Muster RM und die Antigen Spot-Muster AM sich dadurch unterscheiden, dass jeweils Spots unterschiedlicher Größe für die unterschiedlichen jeweiligen Muster RM, AM ausgebildet werden, welches im Zuge eines Herstellungsverfahrens, wie in der Fig. 4a illustriert, beispielsweise durch unterschiedliche Pipettengrößen zur Erzeugung unterschiedlicher Größen von Spots bzw. unterschiedlicher Größen von Tropfen zur Ausbildung der Spots verwendet werden können.

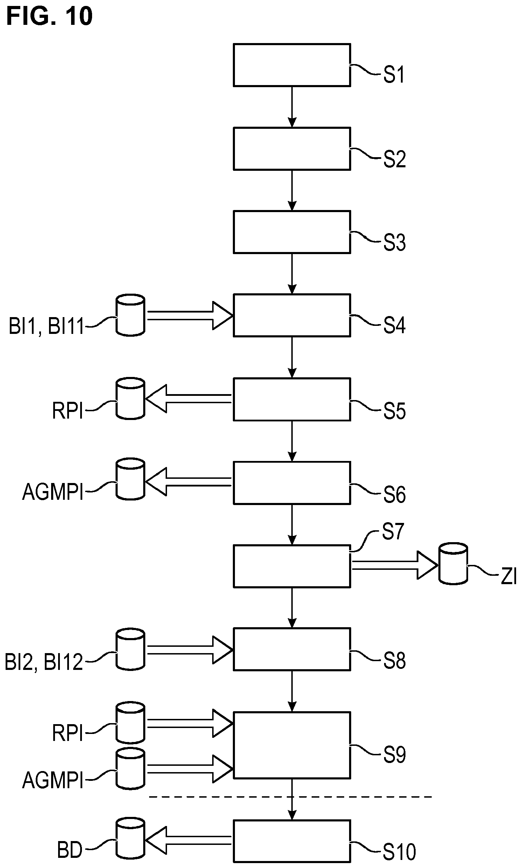

Die Fig. 10 zeigt bevorzugte Schritte eines erfindungsgemäßen Verfahrens zum automatisierten Detektieren von Antikörpern in einer flüssigen biologischen Probe.

In einem Schritt S1 erfolgt ein Bereitstellen eines Antigen-Chips nach einer de zuvor beschriebenen Ausführungsformen. In einem Schritt S2 erfolgt ein Inkubieren der Spots des Antigen-Chips mit der biologischen Probe. In einem Schritt S3 erfolgt ein Inkubieren der Spots mit einem Konjugat, welches einen mit einem zweiten Farbstoff markierten sekundären Antikörper aufweist. In einem Schritt S4 erfolgt ein Bereitstellen oder ein Erfassen einer ersten Bildinformation BI1, BI11, welche eine Färbung von Referenz-Spots und von Antigen-Spots des Antigen-Chips durch einen ersten Farbstoff repräsentiert.

In dem Fall, dass der erste Farbstoff ein Fluoreszenzfarbstoff ist, erfolgt vorzugsweise in dem Schritt S4 ein Beleuchten des Antigen-Chips mit einem ersten Anregungslicht einer ersten Wellenlänge zum Anregen einer Emission von erster Fluoreszenzstrahlung durch den ersten Farbstoff. Die erste Bildinformation repräsentiert dann eine Färbung der Referenz-Spots und der Antigen-Spots des Antigen-Chips durch die erste Fluoreszenzstrahlung. Dieses erfolgt vorzugsweise in einem sogenannten Rotkanal.

In einem Schritt S5 erfolgt ein Detektieren des Referenz-Musters sowie einer zugehörigen Referenz-Position auf der Substratoberfläche unter Annahme einer Anordnungsrichtung der Muster auf der Substratoberfläche auf Basis der wenigstens einen ersten Bildinformation. Die Referenz-Position kann als eine Referenz-Positions-Information RPI bereitgestellt werden.

In einem Schritt S6 erfolgt ein Detektieren der jeweiligen Antigen-Spot-Muster sowie der jeweiligen zugehörigen weiteren Positionen auf der Substratoberfläche unter Annahme einer Anordnungsrichtung der Muster auf der Substratoberfläche auf Basis der wenigstens einen ersten Bildinformation. Dieses kann als eine sogenannte weitere, gesamte Positions-Information AGMPI bezogen auf die weiteren Positionen der Antigen-Spot-Muster bereitgestellt werden.

In einem Schritt S7 wird dann auf Basis der detektierten Referenz-Position sowie der detektierten weiteren Positionen eine Zuordnungs-Information ZI erstellt, welche eine Zuordnung der jeweiligen Antigen-Spot-Muster zu den jeweiligen Antigen-Typen indiziert.

In einem Schritt S8 erfolgt dann ein Erfassen oder ein Bereitstellen einer zweiten Bildinformation BI2, BI12, welche eine potentielle Färbung der Antigen-Spots und vorzugsweise auch der Referenz-Spots durch den zweiten Farbstoff repräsentiert. Im Zuge dieses Schrittes S8 erfolgt in dem Fall, dass der zweite Farbstoff ein Fluoreszenzfarbstoff ist, vorzugsweise ein Beleuchten des Antigen-Chips mit einem zweiten Anregungslicht einer zweiten Wellenlänge, um eine Emission von zweiter Fluoreszenzstrahlung anzuregen. Vorzugsweise ist das zweite Anregungslicht der zweiten Wellenlänge in seinem Wellenlängenbereich identisch zu dem Wellenlängenbereich des ersten Anregungslichtes zur Anregung der ersten Fluoreszenzstrahlung des ersten Fluoreszenzfarbstoffes. Die zweite Bildinformation repräsentiert dann vorzugsweise eine potentielle Färbung der Antigen-Spots des Antigen-Chips durch die zweite Fluoreszenzstrahlung.

In einem Schritt S9 kann dann, vorzugsweise unter Verwendung der Referenz-Positions-Information RPI sowie der weiteren Positions-Information AGMPI, ein Bestimmen jeweiliger Bindungsmaße, welche jeweilige Bindungen bzw. Bindungsgrade von Antikörpern der biologischen Probe an jeweilige Antigentyp Medizin, auf Basis der zweiten Bildinformation BI2, BI12 und der Zuordnungs-Information ZI erfolgen.

Diese Bindungsmaße können vorzugsweise in einem Verfahrenschritt S10 ausgegeben werden, vorzugsweise in Form eines Datenelementes BD über eine Datenschnittstelle.

Die Schritte S4 bis S9 können vorzugsweise in dem erfindungsgemäßen Verfahren für eine automatisierte Bildverarbeitung zum Detektieren von Antikörpern in einer flüssigen biologischen Probe gemäß einer Ausführungsform ausgeführt werden. Gleiches gilt für den vorzugsweise durchzuführenden Schritt S10.

In dem Schritt S10 wird also vorzugsweise über eine Datenschnittstelle eine Information bereitgestellt, welche die Position der Muster und vorzugsweise auch die Position einzelner Antigen-Spots und einzelner Referenz-Spots auf dem Antigen-Chips indiziert.

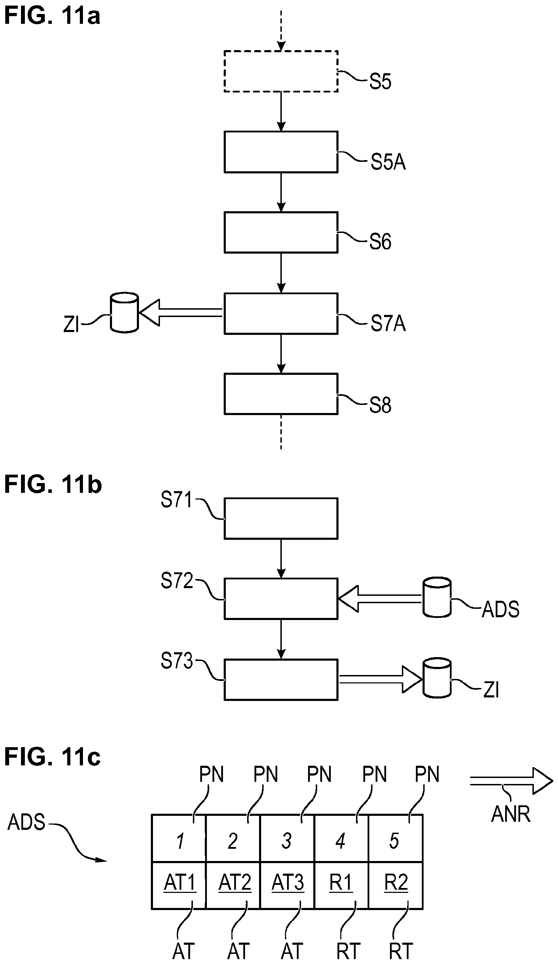

Die in dem Schritt S7 erfolgte Zuordnung von Antigen-Spot-Mustern zu jeweiligen Antigen-Typen erfolgt vorzugsweise in einer Weise, wie in der Fig. 11b illustriert. Hierbei wird in einem Schritt S71 eine räumliche Abfolge der Referenz-Position des Referenz-Musters und der jeweiligen weiteren Positionen der jeweiligen weiteren Antigen-Spot-Muster bestimmt. Diese Position können auch als Positionen entlang einer, insbesondere angenommenen, Anordnungsrichtung der Muster auf der Substratoberfläche bezeichnet werden.

In einem Schritt S72 erfolgt eine Bereitstellung eines Abfolge-Datensatzes ADS, welcher eine Abfolge von Antigen-Typen indiziert. Dieses ist insbesondere eine räumliche Abfolge von Antigen-Typen entlang einer Anordnungsrichtung von Mustern.

Die Fig. 11c illustriert einen beispielhaften Abfolge-Datensatz ADS, welcher für eine Abfolge von Mustern entlang einer Anordnungsrichtung ANR für abfolgende Positionen in entsprechenden Feldern PN jeweilige korrespondierende Antigen-Typen AT1, AT2, AT3 in entsprechenden Feldern AT indiziert. Der Abfolge-Datensatz ADS indiziert also eine räumliche Abfolge von jeweiligen Antigen-Typen für jeweilige Positionen entlang einer Anordnungsrichtung ANR von Spot-Mustern. Ferner werden auch für bestimmte Positionen entsprechende Referenz-Mustertypen R1, R2 in entsprechenden Feldern RT angegeben. In diesem bevorzugten Ausführungsbeispiel ist ein erstes und ein zweites Referenz-Muster vorhanden. Für den Fachmann versteht es sich, dass im Falle nur eines Referenz-Musters nur ein Referenz-Mustertyp R1 vorhanden ist.

In einem Unterschritt S73 aus der Fig. 11b erfolgt dann ein Erstellen einer Zuordnungs-Information ZI, welche die Zuordnung der jeweiligen Antigen-Spot-Muster zu den jeweiligen Antigen-Typen indiziert. Dieses erfolgt auf Basis der detektierten Referenz-Position, der detektierten, jeweiligen weiteren Position der jeweiligen Antigen-Spot-Muster und des Abfolge-Datensatzes ADS.

Vorzugsweise wird im Zuge des Verfahrens auch eine weitere Referenz-Position eines zweiten Referenz-Musters detektiert und im Zuge der Zuordnung der jeweiligen Antigen-Spot-Muster zu den jeweiligen Antigen-Typen berücksichtigt. Dieses ermöglicht eine Kompensation bzw. Berücksichtigung einer unerwünschten 180° Rotation des Antigen-Chips.

Hierzu zeigt die Fig. 11a bevorzugt durchzuführende Schritte. Auf den zuvor beschriebenen Schritt S5 erfolgt dann in einem Schritt S5A ein Detektieren des zweiten Referenz-Musters sowie einer zugehörigen zweiten Referenz-Position auf Basis der ersten Bildinformation. Es folgt dann der zuvor beschriebene Schritt S6. In einem darauffolgenden Schritt S7A erfolgt dann das Erstellen der Zuordnungs-Information ZI, welche die Zuordnung der jeweiligen Antigen-Spot-Muster zu den jeweiligen Antigen-Typen indiziert. Dieses erfolgt auf Basis der detektierten ersten Referenz-Position, der detektierten zweiten Referenz-Position und der detektierten weiteren Position der jeweiligen Antigen-Spot-Muster als auch vorzugsweise des Abfolge-Datensatzes.

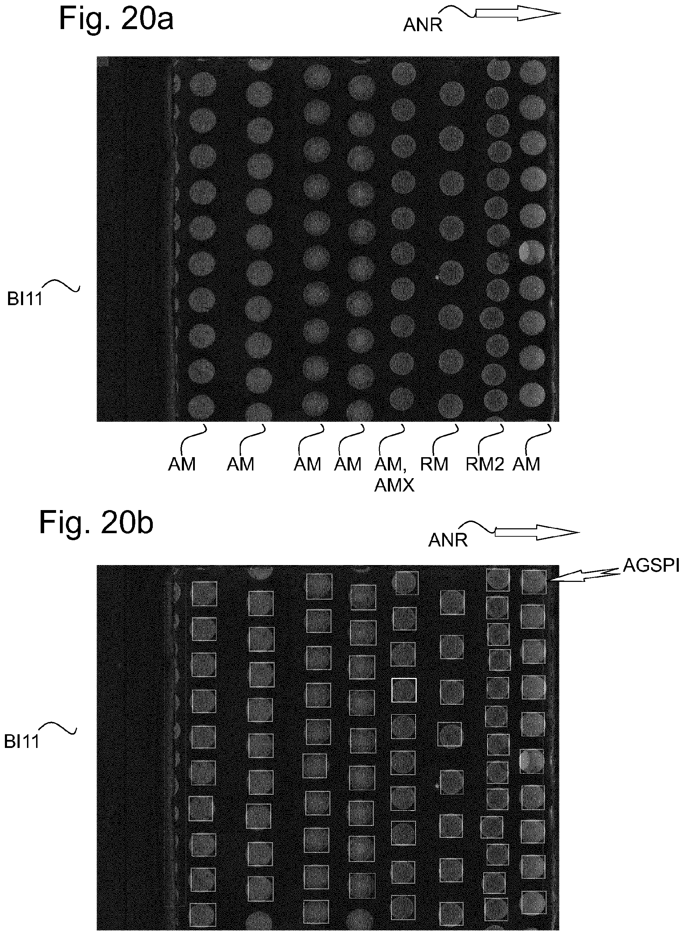

Die Fig. 20a zeigt ein erstes Referenz-Muster RM, ein zweites Referenz-Muster RM2 sowie Antigen-Spot-Muster AM repräsentiert durch eine erste Bildinformation BI11 in einem ersten Farbkanal, welcher vorzugsweise ein Rotkanal ist.

Die Fig. 20b zeigt hierzu in der ersten Bildinformation BI11 entsprechende beispielhafte Antigen-Spot-Positionen AGSPI1, welche als Rechtecke eingezeichnet sind. Nach einer Detektion solcher Antigen-Spot-Positionen AGSPI1 als auch entsprechender Positionen für die Referenz-Muster RM, RM2 aus der Fig. 20a lassen sich dann die einzelnen Spots einzelnen Mustern AM, RM, RM2, insbesondere Linienmustern, zuordnen. Es können so für jeweilige Muster AM, RM, RM2 jeweilige Positionen entlang der Anordnungsrichtung ANR bestimmt werden. Dann können eben in der zuvor beschriebenen Art und Weise vorzugsweise auf Basis eines Abfolge-Datensatzes einzelne Antigen-Spot-Muster einzelnen Antigen-Typen zugeordnet werden.

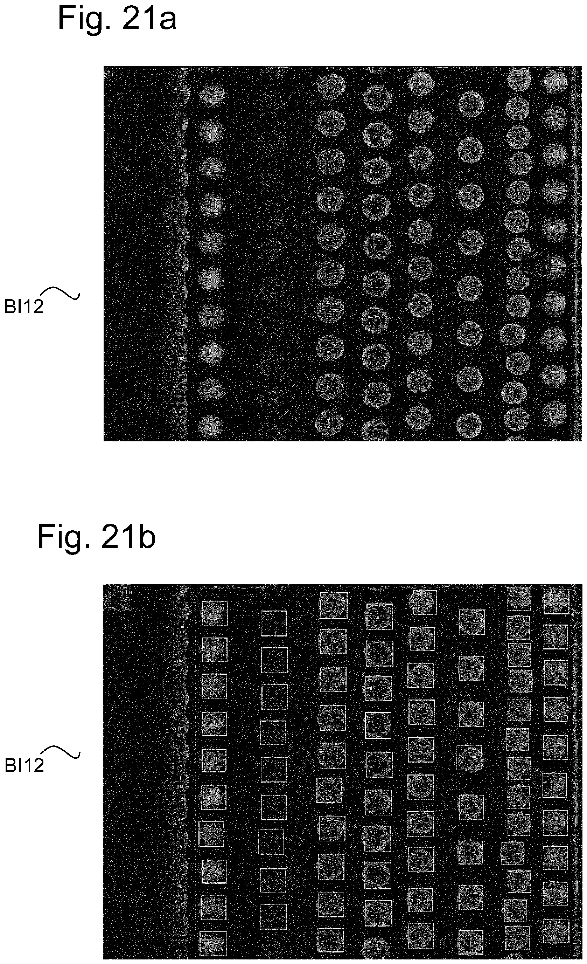

Die Fig. 21a zeigt eine Färbung von Spots durch einen zweiten Farbstoff des gleichen Antigen-Chips wie aus der Fig. 20a und 20b. Die Fig. 21a zeigt eine zweite Bildinformation BI12, welche eine Färbung von Spots, insbesondere der Antigen-Spots durch den zweiten Farbstoff und somit in einem zweiten Farbkanal, vorzugsweise einem Grünkanal, repräsentiert.

Die auf Basis der ersten Bildinformation BI11 aus Fig. 20b erkannten Positionen der Spots können hierbei verwendet werden, um, wie in der Fig. 21b gezeigt, in der zweiten Bildinformation BI21 zu bestimmen, ob für bestimmte Antigen-Spots und somit für bestimmte Antigen-Typen Bindungen von bestimmten Antikörpern aus der biologischen Probe vorliegen. Anhand dieser Bestimmung bzw. Auswertung können dann Bindungsmaße für jeweilige Antigen-Typen bestimmt und vorzugsweise anschließend bereitgestellt werden.

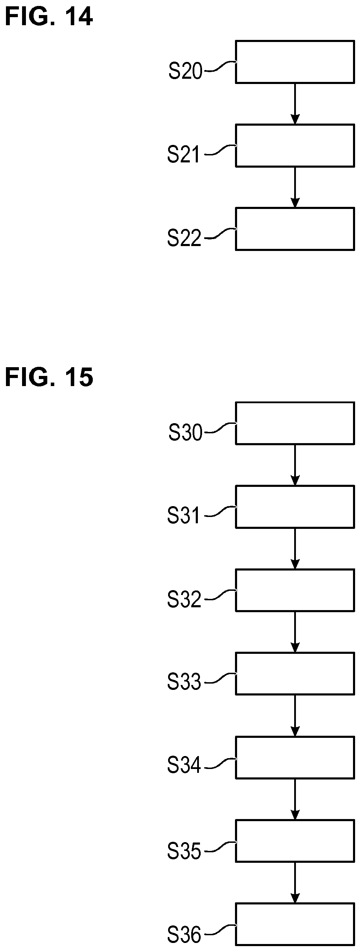

Im Zuge der zuvor unter Bezug auf die Fig. 10 erwähnten Verfahrensschritte S5 und S6 können bevorzugte Teilverfahrensschritte durchgeführt werden, wie in der Fig. 14 eingezeichnet. In einem Verfahrensschritt S20 erfolgt dann ein Erkennen mehrerer potentieller Muster auf Basis der wenigstens einen ersten Bildinformation. In einem weiteren Verfahrensschritt S21 erfolgt dann ein Detektieren jeweiliger regelmäßiger mittlerer Spotabstände von Spots innerhalb eines jeweils erkannten Musters für das jeweils erkannte, potentielle Muster. In einem Schritt S22 erfolgt dann ein Auswählen eines der erkannten Muster als das Referenz-Muster auf Basis der detektierten mittleren Spotabstände. Dieses kann, wie sich für einen Fachmann ergibt, auch für das zweite Referenz-Muster durchgeführt werden.

Es wird nun im Folgenden erläutert, in welcher Weise eine Erkennung von einzelnen Spots und dann eine Erkennung von Mustern bzw. eine Zuordnung von Spots zu Mustern im Detail erfolgen kann. Hierzu zeigt Fig. 15 vorzugsweise durchzuführende Schritte.

Unter Verwendung bzw. auf Basis der ersten Bildinformation BI11 aus der Fig. 20a erfolgt zunächst in einem Schritt S30 mittels einer Hough-Transformation eine Identifikation kreisförmiger Objekte in dem ersten Bild BI11.

In einem Schritt S31 erfolgt für jeden gefundenen Kreis eine Extraktion von Eigenschaften für die identifizierten kreisförmigen Objekte wie z.B. einer Kontur und/oder Intensitätseigenschaften. Die Eigenschaften können ein Umfang eines Kreises, eine Abweichung von einem perfekten freien Kreis oder die durchschnittliche Intensität eines Objektes sein.

In einem Schritt S32 erfolgt dann unter Verwendung einer Support-Vector-Machine eine Klassifikation der vorgeschlagenen kreisförmigen Objekte aus der Hough-Transformation mit deren Eigenschaften in eine erste Klasse "Spot" oder eine zweite Klasse "Bildartefakt". Nur solche Bildbereiche bzw. Objekte, welche als Spot klassifiziert werden, werden im Weiteren verwendet.

Für jeden als Spot klassifizierten Bildbereiche kann dann bereits in dem Schritt S31 eine Position auf dem Antigen-Chip bzw. in der Bildinformation BI11 in X bzw. Y Position bestimmt werden bzw. bestimmt worden sein. In einem Schritt S33 kann dann ein Zusammenfassen von Spots zu einem Muster bzw. einem Linienmuster bzw. einer Linie erfolgen. Hierbei wird eine Liste für die Spots erstellt, welche anhand ihrer Y-Position im Bild sortiert wird. Anschließend wird aus der Liste ein Spot entnommen und sein nächster Nachbar in euklidischer Entfernung ermittelt. Wenn beide Spots in einem Winkel von ca. 90° zueinander liegen bzw. eine Gerade bilden, welche gegenüber der Anordnungsrichtung einen Winkel von 90° bildet, so wird angenommen, dass sie zu einem selben Muster bzw. einer selben Linie gehören. Dieser Wert von 90° kann auch mit einem Unschärfefenster von mehreren Plus- Minusgraden versehen werden. Ist eine solche Zugehörigkeit der zwei betrachteten Spots zueinander zu einem Muster bzw. zu einer solchen Geraden von ca. 90° zur Anordnungsrichtung der Muster nicht gegeben, so wird der nächste nach Entfernung bzw. Y-Position sortierte Spot aus der Liste betrachtet. Können keine Spots aus der Liste mehr dem aktuell betrachteten Muser bzw. der aktuell betrachteten Linie zugeordnet werden, so wird ein neues Muster bzw. eine neue Linie in einer Richtung senkrecht zur Anordnungsrichtung der Muster angefangen. Durch ein solches Verfahren werden dann iterativ die Spots jeweiligen Mustern bzw. jeweiligen Linien zugeordnet.

In einem Schritt S34 erfolgt dann eine Durchnummerierung der Muster bzw. Linienmuster. Dies erfolgt vorzugsweise in der Art, dass das Muster bzw. die Linie, dessen/deren Spots den größten mittleren Abstand zueinander aufweisen, als das Referenz-Muster bzw. als das Referenzlinienmuster angenommen werden. Vorzugsweise wird ein zweites Referenz-Muster als jenes Muster bzw. als jene Linie angenommen, bei welchem/welcher die Spots den geringsten mittleren Abstand zueinander aufweisen.

In einem Schritt S35 können dann auf Basis der zweiten Bildinformation BI12 aus Fig. 21b für jene Spots, welche einem Muster bzw. einer Linie zugeordnet sind, die jeweiligen Intensitäten bzw. Pixel-Intensitäten pro Spot in dem zweiten Farbkanal aufgrund einer Färbung durch den zweiten Farbstoff ermittelt werden. Dieses erfolgt vorzugsweise als der Medianwert der Intensitäten eines Spots.

Abschließend kann in einem Schritt S36 dann eine Bestimmung eines mittleren Intensitätswertes eines Musters in der Weise, dass der Medianwert der Median-Intensitäten der Spots eines Musters ermittelt wird. Dieser Medianwert ist dann ein Bindungsmaß, welches eine Bindung bzw. einen Bindungsgrad von Antikörpern aus der biologischen Probe an einen bestimmten Antigen-Typen indiziert.

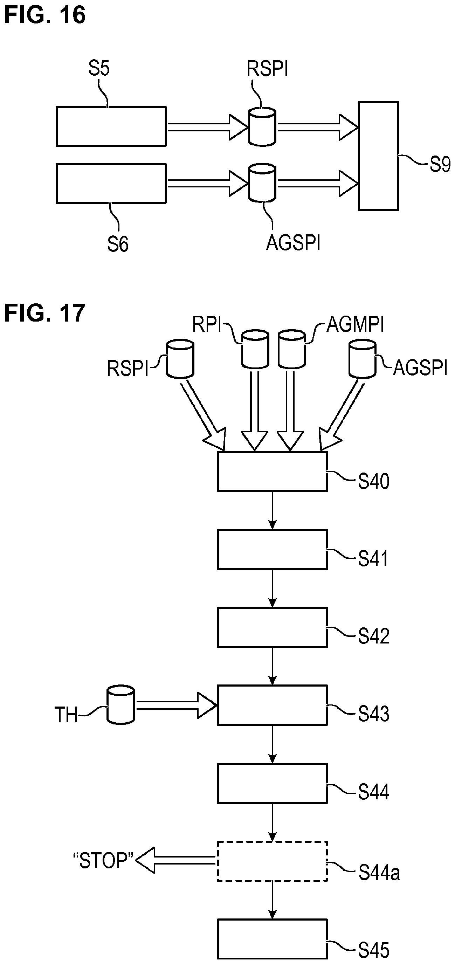

Es können also vorzugsweise im Zuge des Schrittes S9 zum Bestimmen der Bindungsmaße auf Basis der zweiten Bildinformation BI12 die zuvor aus dem Schritt S6 auf Basis der ersten Bildinformation BI11 gewonnene Antigen-Spot-Positions-Information AGSPI sowie die in dem Schritt S5 gewonnene Referenz-Spots-Positions-Information RSPI verwendet werden, wie in der Fig. 16 dargestellt. Das Detektieren eines Referenz-Spots-Musters und der jeweiligen Antigen-Spot-Muster beinhaltet also eine Detektion einzelner Spot-Positionen und eine Zuordnung von Spots bzw. Spotpositionen zu dem Referenz-Muster und den jeweiligen Antigen Spot-Mustern.

Vorzugsweise wird zur Bestimmung eines Bindungsmaßes ein über ein Datenelement bereitgestellter Schwellwert verwendet, welcher auf die für ein Muster bzw. einen Antigentyp ermittelte Medianintensität angewendet werden kann. Unterschreitet der Medianintensitätswert eines Musters den Schwellwert, so wird angenommen, dass gar keine Bindung von Antikörpern vorliegt. Erst in dem Fall, dass der Medianwert eines Musters den vorgegebenen Schwellenwert überschreitet, wird ein Wert ausgegeben, welcher indiziert, dass eine Bindung von Antikörpern an den entsprechenden Antigen-Typen gegeben ist.

Die Ausgabe der Bindungsmaße kann über ein Datenelement erfolgen, bei welchem, wie in Fig. 12 eingezeichnet, ein Datensatz BD eine Zuordnung von Bindungsmaßen BM zu entsprechenden Antigen-Typen AT indiziert. Der Datensatz BD kann beispielsweise eine Liste sein. Für einen den Antigentyp AT1 ist dann ein Bindungsmaß BM1 enthalten, sowie entsprechende Bindungsmaße BM2, BM3 für entsprechende Antigen-Typen AT2, AT3.

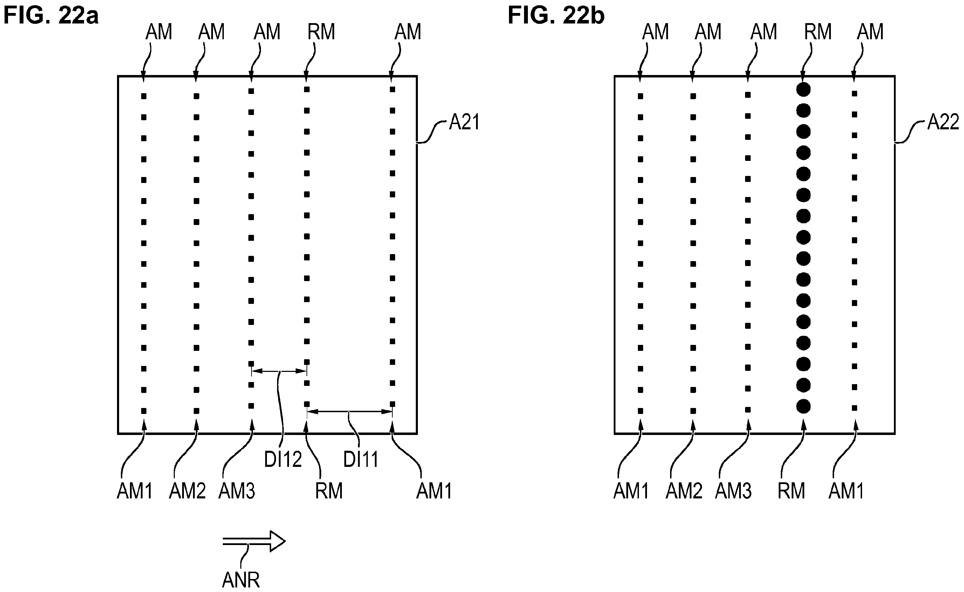

Wie zuvor beschrieben, weist in einer alternativen Ausführungsform das Referenz-Muster zu einem unmittelbar benachbarten Muster entlang der Anordnungsrichtung der Muster einen solchen Nachbar-Abstand auf, welcher sich von den anderen Nachbar-Abständen der Antigen-Spot-Muster unterscheidet. Hierzu zeigt die Fig. 22a eine Ausführungsform A21, bei welcher nur ein Referenz-Muster RM vorhanden ist. Das Referenz-Muster RM weist einen Nachbar-Abstand DI11 zu dem in der Anordnungsrichtung ANR nächsten direkt benachbarten Muster AM1 auf, welcher größer ist als der Nachbar-Abstand DI12 der anderen Muster AM1, AM2, AM3 zu ihren in der Anordnungsrichtung ANR nächsten direkt benachbarten Mustern AM2, AM3, RM.

In dieser Ausführungsform können dann im Zuge des Verfahrens zum automatisierten Detektieren sowie des Verfahrens der automatisierten Bildverarbeitung folgende Schritte vorzugsweise ausgeführt werden:
- Erkennen mehrerer Muster AM1, AM2, AM3, RM auf Basis der ersten Bildinformation;
- Detektieren jeweiliger Nachbar-Abstände DI11, DI12 der erkannten Muster, insbesondere entlang der Anordnungsrichtung der Muster, zu ihren, insbesondere direkt oder unmittelbar, in Richtung der Anordnungsrichtung der Muster jeweils nächsten benachbarten Mustern;
- Auswählen eines der erkannten Muster als das Referenz-Muster auf Basis der detektierten Nachbar-Abstände DI11, DI12.

Hierbei können die, wie in Fig. 20b gezeigt, auf Basis der ersten Bildinformation BI11 detektierten Spot-Positionen der Referenz-Spots und der Antigen-Spots genutzt werden, um, wie zuvor im Detail beschrieben, die Spots jeweiligen Mustern bzw. Linienmustern zuzuordnen, so dass eben mehrere Muster auf Basis der ersten Bildinformation BI11 erkannt werden. Entlang bzw. in der Anordnungsrichtung ANR der Muster können dann jeweilige Nachbar-Abstände DI11, DI12 zwischen den Mustern bestimmt werden, also z.B. ein Nachbar-Abstand für das Referenz-Muster zu dem rechts von ihm liegenden, direkt benachbarten nächsten Muster in Richtung der Anordnungsrichtung ANR. Weist das Referenz-Muster einen Nachbar-Abstand auf, welcher sich von den Nachbarabständen der Antigen-Spot-Mustern unterscheidet, so kann das Referent-Muster sicher detektiert und erkannt werden.

Wie zuvor beschrieben, ist gemäß einer alternativen Ausführungsform vorgesehen, dass innerhalb des Referenz-Musters und innerhalb der Antigen-Spot-Muster die jeweils zugehörigen Spots eine jeweilige, gleiche Größe aufweisen, wobei die Größe der Referenz-Spots des Referenz-Musters sich von den Größen der Antigen-Spots der Antigen-Spot-Muster unterscheidet. Hierzu zeigt Fig. 22b eine bevorzugte Ausführungsform A22 eines Antigen-Chips, bei welcher nur ein Referenz-Muster RM vorhanden ist. Die Referenz-Spots des Referenz-Musters RM weisen eine gleiche Größe auf. Diese Größe unterscheidet sich von der gleichen, gemeinsamen Größe, welche die Antigen-Spots der Antigen-Muster AM aufweisen.

In dieser Ausführungsform können dann im Zuge des Verfahrens zum automatisierten Detektieren sowie des Verfahrens der automatisierten Bildverarbeitung folgende Schritte vorzugsweise ausgeführt werden:
- Erkennen mehrerer Muster RM, AM1, AM2, AM3 auf Basis der wenigstens einen ersten Bildinformation,
- Detektieren jeweiliger Spot-Größen für die jeweiligen erkannten Muster RM, AM1, AM2, AM3;
- Auswahl eines der erkannten Muster als das Referenz-Muster RM auf Basis der detektierten Spot-Größen
Hierbei können die, wie in Fig. 20b gezeigt, auf Basis der ersten Bildinformation BI11 detektierten Spot-Positionen der Referenz-Spots und der Antigen-Spots genutzt werden., um, wie zuvor im Detail beschrieben, die Spots jeweiligen Mustern bzw. Linienmustern zuzuordnen, so dass eben mehrere Muster auf Basis der ersten Bildinformation BI11 erkannt werden. Für jeden Spot wird dann eine Eigenschaft wie der Umfang des identifizierten Kreises bzw. Kreisobjektes bestimmt. Hierdurch können Spots von gleichem Umfang bzw. gleicher Größe dann einem gleichen Muster zugeordnet werden. Jenes Muster, dessen Spots im Mittel die größte Spot-Größe gegenüber den anderen Mustern aufweist, kann dann als das Referenz-Muster detektiert werden.

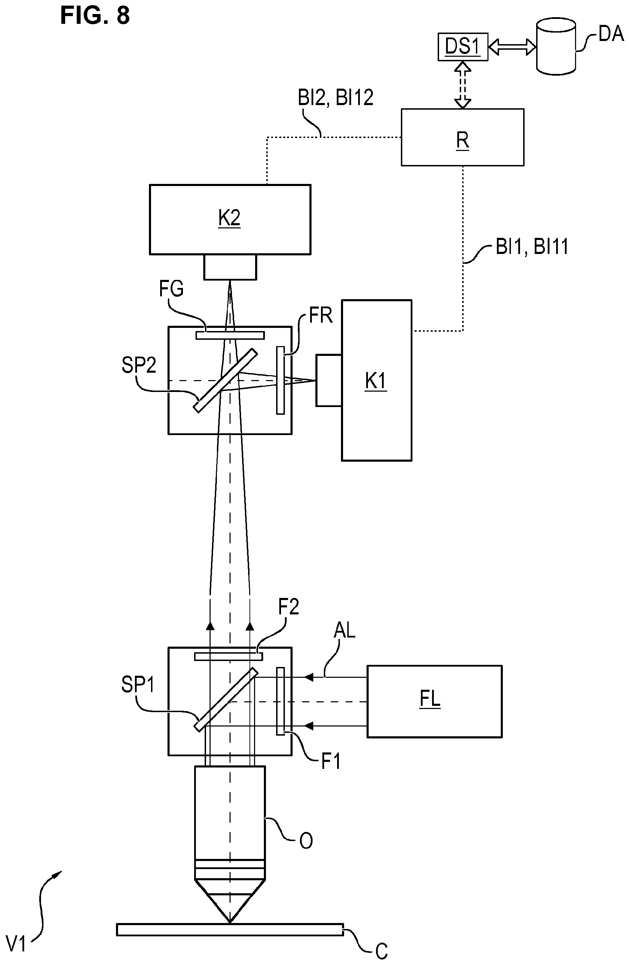

Die Fig. 8 zeigt eine erfindungsgemäße Vorrichtung V1 gemäß einer Ausführungsform zum automatisierten Detektieren von Antikörpern in einer Probe. Die Vorrichtung V1 umfasst eine sogenannte Mikroskopvorrichtung.

Ein Antigen-Chip C kann durch ein Objektiv O mittels wenigstens einer Kamera K1, K2 betrachtet werden.

Die Kameras K1, K2 können als Bilderfassungseinheiten bezeichnet werden. Der Aufbau aus der Fig. 8 für die Vorrichtung V1 sieht eine explizite Trennung zweier Farbkanäle bzw. optischer Kanäle vor einer Erfassung von Bildinformationen durch separate Bilderfassungseinheiten K1, K2 vor. Für den Fachmann ist es offensichtlich, dass durch einen zeitlichen Austausch von optischen Filtern eine solche Vorrichtung auch realisiert werden kann, indem nur eine einzige Bilderfassungseinheit vorgesehen ist aber durch die Verwendung der unterschiedlichen optischen Filter für unterschiedliche Farbkanäle die zuvor beschriebenen jeweiligen Bildinformationen der in jeweiligen Farbkanälen erfasst werden können.

Eine Fluoreszenz-Lichtquelle LQ emittiert ein Anregungslicht AL, welches mittels eines Anregungs-Lichtfilters F1 so gefiltert wird, dass ein in Fig. 7 beschriebenes Anregungslicht-Spektrum AL auf den dichroitischen Spiegel SP1 gerichtet wird. Der dichroitische Spiegel SP1 lenkt dann dieses Anregungslicht AL hin zum Objektiv O, durch welches dann das Anregungslicht auf den Antigen-Chip C und dessen Spots trifft. Von dem Antigen-Chip C bzw. den Fluoreszenzfarbstoffen der Spots emittierte erste und zweite Fluoreszenzstrahlung gelangt dann durch das Objektiv O durch den dichroitischen Spiegel SP1 hindurch hin zu einem optischen Filter F2, welches das Anregungslicht AL bzw. dessen Spektrum blockiert und die Fluoreszenzstrahlung des ersten Farbkanals und auch eines zweiten Farbkanals transmittiert. Die Fluoreszenzstrahlung gelangt dann zu einem weiteren dichroitischen Spiegel SP2, welcher Fluoreszenzstrahlung des ersten Farbkanals bzw. erste Fluoreszenzstrahlung zu einer ersten Bilderfassungseinheit bzw. Kamera K1 reflektiert. Vorzugsweise wird durch einen optischen Filter FR noch eine Filterung der ersten Fluoreszenzstrahlung aus dem eintretenden Spektrum durchgeführt. Der Filter FR ist insbesondere ein Rotkanalfilter.

Der dichroitische Spiegel SP2 transmittiert die zweite Fluoreszenzstrahlung bzw. Licht des zweiten Farbkanals hin zu einer zweiten Bilderfassungseinheit bzw. Kamera K2. Vorzugsweise wird durch einen weiteren optischen Filter FG diese Strahlung nochmals gefiltert, wobei der optische Filter FG insbesondere ein Grünkanalfilter ist.

Die erste Kamera K1 erfasst dann die erste Bildinformation BI1 und stellt diese an die Recheneinheit R bereit. Die zweite Bilderfassungseinheit K2 erfasst dann die zweite Bildinformation BI2 und stellt diese an die Recheneinheit R2 bereit.

Die erste Bilderfassungseinheit und die zweite Bilderfassungseinheit als ein Beispiel für wenigstens eine Bilderfassungseinheit sind dann also ausgebildet zum Erfassen einer ersten Bildinformation BI1, welche eine Färbung von Referenz-Spots und von Antigen-Spots eines Antigen-Chips C durch einen ersten Farbstoff, insbesondere einen ersten Fluoreszenzfarbstoff, repräsentiert. Ferner ist die wenigstens eine Bilderfassungseinheit K1, K2 ausgebildet zum Erfassen einer zweiten Bildinformation BI2, welche eine potentielle Färbung der Antigen-Spots des Antigen-Chips durch einen zweiten Farbstoff, insbesondere einen zweiten Fluoreszenzfarbstoff, nach Inkubieren des Antigen-Chips mit der biologischen Probe und mit einem Konjugat, welches eine mit dem zweiten Farbstoff markierten sekundären Antikörper aufweist, repräsentiert.

Die Recheneinheit R ist insbesondere ausgebildet, einen oder mehrere der zuvor unter Bezug auf das Verfahren zum automatisierten Detektieren von Antikörpern sowie das Verfahren einer automatisierten Bildverarbeitung zum Detektieren von Antikörpern beschriebenen Schritte S4, S5, S6, S7 sowie S9 aus der Fig. 10 durchzuführen. Hierzu steuert die Recheneinheit R die Bilderfassungseinheiten K1, K2 sowie die Fluoreszenz-Lichtquelle LQ entsprechend an.

Die Vorrichtung V1 aus in Fig. 8 weist ferner vorzugsweise eine Datenschnittstelle DS1 auf, über welche Datenelemente DA entgegengenommen oder ausgesendet werden können. Insbesondere können hier über solche Datenelemente DA Bindungsmaße übermittelt werden als auch vorzugsweise Datenelemente entgegengenommen werden, welche einen Schwellwert indizieren.

Über die Datenschnittstelle DS1 kann die Vorrichtung V1 Datenelemente DA austauschen. Solche Datenelemente DA können dann eines oder mehrere der folgenden Datenelemente sein:
- ein entgegenzunehmender Schwellwertdatenelement TH aus Fig. 17,
- ein entgegenzunehmender Abfolge-Datensatz ADS aus Fig. 11c
- ein auszugebender bzw. bereitzustellender Bindungsdatensatz BD aus Fig. 10,
- eine auszugebende bzw. bereitzustellende Zuordnungs-Information ZI aus Fig. 11a.

Die Vorrichtung V1 weist also mit der Fluoreszenz-Lichtquelle LQ eine Beleuchtungseinheit zum Aussenden von Anregungslicht zum Anregen einer Emission von erster Fluoreszenzstrahlung eines ersten Wellenlängenbereiches durch den ersten Farbstoffe auf. Ferner stellt die Lichtquelle LQ auch wenigstens eine Beleuchtungseinheit zum Aussenden von Anregungslicht zum Anregen einer Emission von zweiter Fluoreszenzstrahlung eines zweiten Wellenlängenbereiches durch den zweiten Farbstoff dar.

Die Fig. 17 zeigt bevorzugte Schritte im Zuge des beschriebenen Verfahrens zum automatisierten Detektieren von Antikörpern und/oder des Verfahrens für eine automatisierte Bildverarbeitung.

In einem Schritt S40 erfolgt, wie zuvor beschrieben, in dem zweiten Farbkanal bzw. der zweiten Bildinformation BI12 aus Fig. 21a bzw. 21b eine Identifikation von Antigen-Spots unter Verwendung der Antigen-Spot-Positions-Informationen AGSPI bzw. der weiteren Positions-Information AGMPI. Ferner erfolgt vorzugsweise in dem Fall, dass die Referenz-Spots auch ein Antigen aufweisen, eine Verwendung der Referenz-Positions-Informationen RPI bzw. der Referenz-Spots-Positions-Information RSPI eine Identifikation der Referenz-Spots.

In der zuvor beschriebenen Art und Weise erfolgt in dem Schritt S41 eine Zuordnung von Bildbereichen bzw. Spots zu bestimmten Mustern und somit auch zu bestimmten Antigen-Typen.

In einem Schritt S42 erfolgt eine Bestimmung einer mittleren Intensität bzw. Medianintensität eines Musters in dem zweiten Farbkanal bzw. auf Basis der zweiten Bildinformation.

In dem Verfahrensschritt S43 erfolgt ein Bereitstellen bzw. Entgegennehmen eines Datensatzes TH, welcher einen auf Medianintensitäten anzuwendenden Schwellenwert indiziert.

Es erfolgt dann in dem Schritt S43 eine Anwendung dieses Schwellenwertes auf die ermittelten mittleren Intensitäten der jeweiligen Muster.

In einem Schritt S44 erfolgt dann eine Bestimmung jeweiliger Bindungsmaße für die jeweiligen Muster.

In einem vorzugsweise durchzuführenden Schritt S44a erfolgt dann eine Ausgabe eines Hinweises an den Nutzer, vorzugsweise mittels einer optischen Anzeigeeinheit oder vorzugsweise mittels eines über eine Datenschnittstelle auszugebenden Datenelementes, in dem Fall, dass die Referenz-Spots des Referenz-Musters in dem zweiten Farbkanal bzw. der zweiten Bildinformation keine hinreichende Färbung bzw. mittlere Intensität aufweisen. Dieses erfolgt insbesondere in dem Fall, dass die Referenz-Spots des Referenz-Musters einen Antikörper, insbesondere IgG, aufweisen, mittels dessen kontrolliert werden kann, ob eine ordnungsgemäße Inkubation des Antigen-Chips mit einer flüssigen biologischen Probe eines humanen Patienten erfolgte.

In dem Schritt S45 erfolgt dann die Ausgabe der Bindungsmaße.

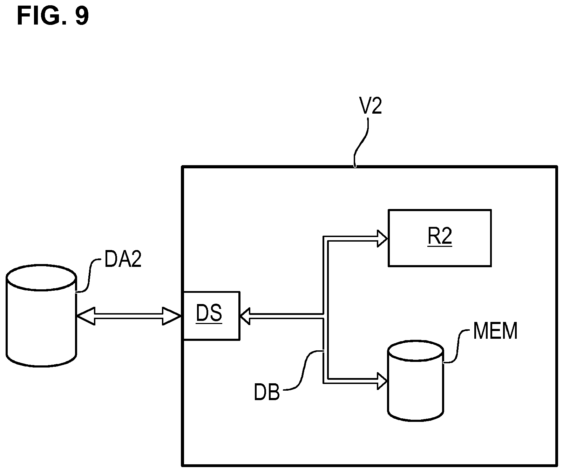

Die Fig. 9 zeigt eine Ausführungsform einer erfindungsgemäßen Datennetzwerkvorrichtung V2, welche vorzugsweise auch als Cloudsystem bezeichnet werden kann.

Die Datennetzwerkvorrichtung V2 zur automatisierten Bildauswertung zum Detektieren von Antikörpern in einer flüssigen und biologischen Probe weist wenigstens eine Datenschnittstelle DS auf, über welche Datenelemente DA2 über ein Datennetzwerk ausgetauscht werden können.

Die Vorrichtung V2 weist wenigstens eine Recheneinheit R2 auf, welche vorzugsweise über einen Datenbus DB mit der Datenschnittstelle DS verbunden ist. Vorzugsweise weist die Datennetzvorrichtung V2 wenigstens eine Speichereinheit MEM auf.

Die wenigstens eine Datenschnittstelle DS ist ausgebildet zum Entgegennehmen einer ersten Bildinformation, welche eine Färbung von Referenz-Spots und von Antigen-Spots eines Antigen-Chips durch einen ersten Farbstoff repräsentiert. Die wenigstens eine Datenschnittstelle DS ist ferner ausgebildet zum Entgegennehmen einer zweiten Bildinformation, welche eine potentielle Färbung der Antigen-Spots des Antigen-Chips durch einen zweiten Farbstoff nach Inkubieren des Antigen-Chips mit der biologischen Probe und einem Konjugat, welches ein mit dem zweiten Farbstoff markierten sekundären Antikörper aufweist, repräsentiert.

Die Recheneinheit R2 ist ausgebildet, die zuvor unter Bezug auf das Verfahren für eine automatisierte Bildverarbeitung beschriebenen Schritte durchzuführen, insbesondere einen oder mehrere der Schritte S4, S5, S6, S7 und S9. Ferner ist die Recheneinheit R2 ausgebildet, weitere vorzugsweise durchzuführende Schritte des Verfahrens zur automatisierten Bildverarbeitung durchzuführen, welche zuvor im Detail dargelegt wurden.

Die Vorrichtung V2 aus der Fig. 9 ist ferner vorzugsweise ausgebildet, über die Datenschnittstelle DS Datenelemente DA2 bereitzustellen, welche eine Information darstellen, welche die jeweiligen Bindungsmaße von Antikörpern an die jeweiligen Antigen-Typen indizieren.

Ferner ist die Datenschnittstelle DS ferner ausgebildet, um über Datenelemente detektierte Positionen von Antigen-Spots und/oder Mustern sowie Referenz-Spots oder Referenz-Mustern zu indizieren.

In einem Verfahren, welches die Vorrichtung V2 aus der Fig. 9 durchführt, erfolgt eine automatisierte Bildverarbeitung zum Detektieren von Antikörpern in einer flüssigen biologischen Probe insbesondere dadurch, dass die erste Bildinformation über die wenigstens eine Datenschnittstelle DS entgegengenommen wird und mittels der wenigstens eine Speichereinheit MEM bereitgestellt wird, wobei ferner die zweite Bildinformation über die Datenschnittstelle DS entgegengenommen wird und mittels der Speichereinheit MEM ebenfalls bereitgestellt wird.

Vorzugsweise ist die Vorrichtung V2 ausgebildet, über die Datenschnittstelle DS ein Datenelement entgegenzunehmen, welches bestimmte Antigen-Typen indiziert, zu welchen Bindungsmaße ausgegeben werden sollen. Es erfolgt dann vorzugsweise eine Auswertung für diese indizierten bestimmten Antigen-Typen und auch eine Übermittlung eines Datenelementes, welche Bindungsmaße der entsprechenden bestimmten Antigen-Typen indiziert.

Obwohl manche Aspekte im Zusammenhang mit einer Vorrichtung beschrieben wurden, versteht es sich, dass diese Aspekte auch eine Beschreibung der entsprechenden Verfahren darstellen, sodass ein Block oder ein Bauelement einer Vorrichtung auch als ein entsprechender Verfahrensschritt oder als ein Merkmal eines Verfahrensschrittes zu verstehen ist. Analog dazu stellen Aspekte, die im Zusammenhang mit einem oder als ein Verfahrensschritt beschrieben wurden, auch eine Beschreibung eines entsprechenden Blocks oder Details oder Merkmals einer entsprechenden Vorrichtung dar.

Je nach bestimmten Implementierungsanforderungen können Ausführungsbeispiele der Erfindung die Recheneinheiten R, R2 in Hardware und/oder in Software umsetzen. Eine Umsetzung einer hier genannten Recheneinheit R, R2 kann hier als wenigstens eine Recheneinheit erfolgen oder aber durch mehrere Recheneinheiten im Verbund. Die Implementierung kann unter Verwendung eines digitalen Speichermediums, beispielsweise einer Floppy-Disk, einer DVD, einer Blu-Ray Disc, einer CD, eines ROM, eines PROM, eines EPROM, eines EEPROM oder eines FLASH-Speichers, einer Festplatte oder eines anderen magnetischen oder optischen Speichers durchgeführt werden, auf dem elektronisch lesbare Steuersignale gespeichert sind, die mit einer programmierbaren Hardwarekomponente derart zusammenwirken können oder zusammenwirken, dass das jeweilige Verfahren durchgeführt wird.

Eine programmierbare Hardwarekomponente kann als Recheneinheit durch einen Prozessor, einen Computerprozessor (CPU = Central Processing Unit), einen Computer, ein Computersystem, einen anwendungsspezifischen integrierten Schaltkreis (ASIC = Application-Specific Integrated Circuit), einen integrierten Schaltkreis (IC = Integrated Circuit), ein Ein-Chip-System (SOC = System on Chip), ein programmierbares Logikelement oder ein feldprogrammierbares Gatterarray mit einem Mikroprozessor (FPGA = Field Programmable Gate Array) gebildet sein.

Das digitale Speichermedium kann daher maschinen- oder computerlesbar sein. Manche Ausführungsbeispiele umfassen also einen Datenträger, der elektronisch lesbare Steuersignale aufweist, die in der Lage sind, mit einem programmierbaren Computersystem oder einer programmierbare Hardwarekomponente derart zusammenzuwirken, dass eines der hierin beschriebenen Verfahren durchgeführt wird

Allgemein können Ausführungsbeispiele oder Teile der Ausführungsbeispiele der vorliegenden Erfindung als Programm, Firmware, Computerprogramm oder Computerprogrammprodukt mit einem Programmcode oder als Daten implementiert sein, wobei der Programmcode oder die Daten dahin gehend wirksam ist bzw. sind, eines der Verfahren oder ein Teil eines Verfahrens durchzuführen, wenn das Programm auf einem Prozessor oder einer programmierbaren Hardwarekomponente abläuft.

## Patentansprüche

1. Antigen-Chip (A2, A11, A12, A21, A22),
aufweisend eine ebene Substratoberfläche (SO) mit darauf aufgebrachten, zueinander beabstandeten Antigen-Spots (AS), welche einen gleichen, gemeinsamen Farbstoff aufweisen,
wobei die Antigen-Spots (AS) jeweilige Antigen-Spot-Mengen (AG) ausbilden, welche korrespondierende jeweilige, regelmäßige Antigen-Spot-Muster (AM, AM1, AM2) ausbilden,
wobei Antigen-Spots (AS) einer gleichen Antigen-Spot-Menge einen gleichen, gemeinsamen Antigentyp aufweisen und wobei zwei oder mehr der Antigen-Spot-Mengen (A1, A2) unterschiedliche Antigentypen aufweisen,
**dadurch gekennzeichnet, dass** auf der Substratoberfläche (SO) ferner Referenz-Spots (RS) aufgebracht sind, welche eine Referenz-Spot-Menge ausbilden, welche ein regelmäßiges Referenz-Muster (RM) ausbildet,
dass ferner sich das Referenz-Muster von den Antigen-Spot-Mustern hinsichtlich seiner Regelmäßigkeit unterscheidet,
wobei die Referenz-Spots (RS) ebenfalls den gleichen, gemeinsamen Farbstoff aufweisen.

2. Antigen-Chip nach Anspruch 1 (A2, A11, A12, A21, A22),
**dadurch gekennzeichnet, dass** sich das Referenz-Muster (RM) hinsichtlich seiner Regelmäßigkeit von den Antigen-Spot-Mustern (AM, AM1, AM2) durch eines oder mehrere der folgenden Kriterien unterscheidet:
o innerhalb des Referenz-Musters (RM) und der innerhalb der Antigen-Spot-Muster (AM, AM1, AM2) folgen die jeweils zugehörigen Spots in jeweiligen regelmäßigen Abständen (SD1, SD2) aufeinander, wobei der regelmäßige Abstand (SD1) der Referenz-Spots (RS) des Referenz-Musters (RM) sich von den regelmäßigen Abständen (SD2) der Antigen-Spots der Antigen-Spot-Muster (AM, AM1, AM2) unterscheidet;
o das Referenz-Muster (RM) weist zu einem unmittelbar benachbarten Muster einen solchen Nachbar-Abstand auf, welcher sich von den anderen Nachbar-Abständen der Antigen-Spot-Muster unterscheidet;
o innerhalb des Referenz-Musters (RM) und innerhalb der Antigen-Spot-Muster (AM, AM1, AM2) weisen die jeweils zugehörigen Spots eine jeweilige, gleiche Größe auf, so dass die Größe der Referenz-Spots des Referenz-Musters (RM) sich von den Größen der Antigen-Spots der Antigen-Spot-Muster (AM, AM1, AM2) unterscheidet.

3. Antigen-Chip (A2, A11, A12, A21, A22) nach Anspruch 1 oder 2,
**dadurch gekennzeichnet, dass** das Referenzmuster (RM) und die Antigenmuster (AM, AM1, AM2) entlang einer Anordnungsrichtung (ANR) der Muster angeordnet sind,
und dass das Referenzmuster (RM) und die Antigenmuster (AM, AM1, AM2) jeweilige Ausbreitungsrichtungen (ABR) aufweisen, welche im Wesentlichen senkrecht zu der Anordnungsrichtung (ANR) der Muster (RM, AM, AM1, AM2) verläuft.

4. Antigen-Chip (A2, A11, A12, A21, A22) nach Anspruch 1 oder 2,
**dadurch gekennzeichnet, dass** die Referenz-Spots der Referenzspot-Menge ein Referenz-Linienmuster (RM) ausbilden und dass ferner die jeweiligen Antigen-Spots der jeweiligen Antigen-Spot-Mengen jeweilige Antigen-Linienmuster (AM, AM1, AM2) ausbilden,
wobei die Linienmuster im Wesentlichen parallel zueinander verlaufen.

5. Antigen-Chip (A2, A11, A12, A21, A22) nach Anspruch 1,
**dadurch gekennzeichnet, dass** der gleiche, gemeinsame Farbstoff ein Fluoreszenzfarbstoff ist.

6. Antigen-Chip (A2, A11, A12, A21, A22) nach Anspruch 1,
**dadurch gekennzeichnet, dass** der zweite Farbstoff ein Fluoreszenzfarbstoff ist.

7. Antigen-Chip (A2, A11, A12, A21, A22) nach einem der Ansprüche 1 bis 6,
**dadurch gekennzeichnet, dass** die Referenz-Spots (RS) der Referenz-Spot-Menge den Farbstoff in einer Farbstoffkonzentration aufweisen, welche größer ist als die Farbstoffkonzentrationen, in welchen die Antigen-Spots der Antigen-Spot-Mengen (AM, AM1, AM2) den Farbstoff aufweisen.

8. Antigen-Chip (A2, A11, A12) nach einem der Ansprüche 1 bis 5,
wobei die Referenz-Spots (RS) erste Referenz-Spots sind, welche eine erste Referenz-Spot-Menge ausbilden, welche wiederum ein erstes regelmäßiges Referenz-Muster (RM) ausbildet,
**gekennzeichnet durch** zweite Referenz-Spots, welche eine zweite Referenz-Spot-Menge ausbilden, welche wiederum ein zweites regelmäßiges Referenz-Muster (RM2) ausbildet, wobei die zweiten Referenz-Spots ebenfalls den gleichen, gemeinsamen Farbstoff aufweisen,
ferner **dadurch gekennzeichnet, dass** sich das zweite Referenz-Muster (RM2) von den Antigen-Spot-Mustern (AM, AM1, AM2) der Antigen-Spot-Mengen und zusätzlich auch von dem ersten Referenz-Muster (RM) der ersten Referenz-Spots hinsichtlich seiner Regelmäßigkeit unterscheidet.

9. Antigen-Chip nach einem der Ansprüche 1 bis 6,
wobei die Referenz-Spots (RS) ferner ein Antigen aufweisen.

10. Antigen-Chip nach einem der Ansprüche 1 bis 6,
wobei die Referenz-Spots (RS) ferner einen Antikörper, insbesondere IgG, aufweisen.

11. Verfahren zum automatisierten Detektieren von Antikörpern in einer flüssigen biologischen Probe mittels Verwendung eines Antigen-Chips (A2, A11, A12, A21, A22), aufweisend die Schritte
Bereitstellen eines Antigen-Chips (A2, A11, A12, A21, A22) nach einem der Ansprüche 1 bis 10, (S1)
Inkubieren der Spots des Antigen-Chips (A2, A11, A12, A21, A22) mit der biologischen Probe, (S2)
Inkubieren der Spots mit einem Konjugat, welches einen mit einem zweiten Farbstoff markierten sekundären Antikörper aufweist, (S3)
Erfassen oder Bereitstellen wenigstens einer ersten Bildinformation (BI1, BI11), welche eine Färbung der Referenz-Spots und der Antigen-Spots durch den ersten Farbstoff repräsentiert, (S4)
Detektieren des Referenzmusters (RM) sowie einer zugehörigen Referenz-Position auf Basis der wenigstens einen ersten Bildinformation (BI1, BI11), (S5)
Detektieren der jeweiligen Antigen-Spot-Muster (AM, AM1, AM2) sowie jeweiliger zugehöriger weiterer Positionen auf Basis der wenigstens einen ersten Bildinformation (BI1, BI11), (S6)
auf Basis der detektierten Referenz-Position und der detektierten weiteren Positionen, Erstellen einer Zuordnungs-Information (ZI), welche eine Zuordnung der jeweiligen Antigen-Spot-Muster (AM, AM1, AM2) zu jeweiligen Antigen-Typen indiziert, (S7)
Erfassen oder Bereitstellen einer zweiten Bildinformation (BI2, BI12), welche eine potentielle Färbung der Antigen-Spots durch den zweiten Farbstoff repräsentiert, (S8)
auf Basis der zweiten Bildinformation (BI2, BI12) und der Zuordnungs-Information (ZI), Bestimmen jeweiliger Bindungsmaße, welche jeweilige Bindungen von Antikörpern der biologischen Probe an jeweilige Antigen-Typen indizieren. (S9)

12. Verfahren für eine automatisierte Bildverarbeitung zum Detektieren von Antikörpern in einer flüssigen biologischen Probe, aufweisend
Bereitstellen oder Erfassen einer ersten Bildinformation (BI1, BI11), welche eine Färbung von Referenz-Spots und von Antigen-Spots eines Antigen-Chips (A2, A11, A12, A21, A22) nach einem der Ansprüche 1 bis 10 durch einen ersten Farbstoff repräsentiert, (S4)
Detektieren des Referenzmusters (RM) sowie einer zugehörigen Referenz-Position, (S5)
Detektieren der jeweiligen Antigen-Spot-Muster (AM, AM1, AM2) sowie jeweiliger zugehöriger weiterer Positionen auf Basis der wenigstens einen ersten Bildinformation (BI1, BI11), (S6)
auf Basis der detektierten Referenz-Position und der detektierten weiteren Positionen, Erstellen einer Zuordnungsinformation (ZI), welche eine Zuordnung der jeweiligen Antigen-Spot-Muster zu jeweiligen Antigen-Typen indiziert, (S7) Bereitstellen oder Erfassen einer zweiten Bildinformation (BI2, BI12), welche eine potentielle Färbung der Antigen-Spots des Antigen-Chips (A2, A11, A12, A21, A22) durch einen zweiten Farbstoff nach Inkubieren der Spots des Antigen-Chips mit der biologische Probe und mit einem Konjugat, welches einen mit dem zweiten Farbstoff markierten sekundären Antikörper aufweist, repräsentiert, (S8)
auf Basis der zweiten Bildinformation (BI2, BI12), Bestimmen jeweiliger Bindungsmaße, welche jeweilige Bindungen von Antikörpern der biologischen Probe an jeweilige Antigen-Typen indizieren. (S9)

13. Verfahren nach Anspruch 11 oder 12,
wobei der Antigen-Chip ein Antigen-Chip nach Anspruch 8 ist,
wobei die Referenz-Position eine erste Referenz-Position ist,
ferner aufweisend:
Detektieren des zweiten Referenzmusters (RM2) sowie einer zweiten zugehörigen Referenz-Position auf Basis der ersten Bildinformation (BI1, BI11),
Erstellen der Zuordnungsinformation (ZI), welche die Zuordnung der jeweiligen Antigen-Spot-Muster zu den jeweiligen Antigen-Typen indiziert, auf Basis der detektierten ersten Referenz-Position, der detektierten zweiten Referenz-Position und der detektierten weiteren Positionen.

14. Verfahren nach Anspruch 11 oder 12,
wobei die Zuordnung der jeweiligen detektierten Antigen-Spot-Muster zu jeweiligen Antigen-Typen erfolgt durch
Bestimmen einer räumlichen Abfolge der Referenz-Position und der weiteren Positionen,
Bereitstellen eines Abfolge-Datensatzes (ADS), welcher eine Abfolge von Antigen-Typen indiziert,
Erstellen der Zuordnungsinformation (ZI), welche die Zuordnung der jeweiligen Antigen-Spot-Muster zu den jeweiligen Antigen-Typen indiziert, auf Basis der detektierten Referenz-Position, der detektierten weiteren Positionen und des Abfolge-Datensatzes (ADS).

15. Verfahren nach Anspruch 12,
wobei die erste Bildinformation (BI1, BI11) über wenigstens eine Datenschnittstelle (DS) entgegengenommen wird und mittels wenigstens einer Speichereinheit (MEM) bereitgestellt wird
und wobei die zweite Bildinformation (BI2, BI12) über die Datenschnittstelle (DS) entgegengenommen wird und mittels der Speichereinheit (MEM) bereitgestellt wird.

16. Verfahren zur Herstellung eines Antigen-Chips (A2, A11, A12, A21, A22) zur Immunodiagnostik, aufweisend
o Bereitstellen eines Substrats mit einer ebenen Substratoberfläche (SO),
o Aufbringen von Referenz-Spots (RS) auf die ebene Substratoberfläche (SO), sowie
o Aufbringen von Antigen-Spots (AS) auf die ebene Substratoberfläche (SO),
wobei das Aufbringen der Referenz-Spots und der Antigen-Spots (AS) derart durchgeführt wird, dass die Referenz-Spots (RS) und die Antigen-Spots (AS) des Antigen-Chips (A2, A11, A12, A21, A22) gemäß einem Antigen-Chip nach einem der Ansprüche 1 bis 10 ausgeführt sind.

17. Verfahren nach Anspruch 16,
ferner aufweisend : Fragmentieren des Substrats (SU), um mehrere Antigen-Chips (A11, A12) zu erhalten.

18. Verfahren nach Anspruch 16 oder 17,
wobei die Substratoberfläche (SO) eine Oberfläche eines Glas-Substrates oder eines mit einer Membran und/oder Folie beschichteten Glas-Substrates ist.

19. Vorrichtung (V1) für eine automatisierte Bildverarbeitung zum Detektieren von Antikörpern in einer flüssigen biologischen Probe, aufweisend
wenigstens eine Bilderfassungseinheit (K1, K2)
o zum Erfassen einer ersten Bildinformation (BI1, BI11), welche eine Färbung von Referenz-Spots (RS) und von Antigen-Spots (AS) eines Antigen-Chips (A2, A11, A12, A21, A22) nach einem der Ansprüche 1 bis 10 durch einen ersten Farbstoff repräsentiert,
o sowie ferner zum Erfassen einer zweiten Bildinformation (BI2, BI12), welche eine potentielle Färbung der Antigen-Spots (AS) des Antigen-Chips (A2, A11, A12, A21, A22) nach einem der Ansprüche 1 bis 10 durch einen zweiten Farbstoff nach Inkubieren der Spots des Antigen-Chips mit der biologische Probe und mit einem Konjugat, welches einen mit dem zweiten Farbstoff markierten sekundären Antikörper aufweist, repräsentiert,
sowie ferner wenigstens eine Recheneinheit (R), welche ausgebildet ist,
o auf Basis der wenigstens einen ersten Bildinformation (BI1, BI11) das Referenzmuster (RM) sowie eine zugehörige Referenz-Position zu detektieren,
o auf Basis der wenigstens einen ersten Bildinformation (BI1, BI11) die jeweiligen Antigen-Spot-Muster (AM) sowie jeweilige zugehörige weitere Positionen zu detektieren,
∘ auf Basis der detektierten Referenz-Position und der detektierten weiteren Positionen eine Zuordnungsinformation (ZI) zu erstellen, welche eine Zuordnung der jeweiligen Antigen-Spot-Muster zu jeweiligen Antigen-Typen indiziert,
∘ sowie auf Basis der zweiten Bildinformation (BI2, BI12) jeweilige Bindungsmaße zu bestimmen, welche jeweilige Bindungen von Antikörpern der biologischen Probe an jeweilige Antigen-Typen indizieren.

20. Vorrichtung nach Anspruch 19,
ferner aufweisend
- wenigstens eine Beleuchtungseinheit (FL) zum Aussenden von Anregungslicht (AL) zum Anregen einer Emission von erster Fluoreszenzstrahlung eines ersten Wellenlängenbereiches durch den ersten Farbstoff.

21. Vorrichtung nach Anspruch 19,
ferner aufweisend
- wenigstens eine Beleuchtungseinheit (FL) zum Aussenden von Anregungslicht (AL) zum Anregen einer Emission von zweiter Fluoreszenzstrahlung eines zweiten Wellenlängenbereiches durch den zweiten Farbstoff.

22. Datennetzwerkvorrichtung (V2) für eine automatisierte Bildverarbeitung zum Detektieren von Antikörpern in einer flüssigen biologischen Probe, aufweisend
wenigstens eine Datenschnittstelle (DS)
o zum Entgegennehmen einer ersten Bildinformation (BI1, BI11), welche eine Färbung von Referenz-Spots (RS) und von Antigen-Spots (AS) eines Antigen-Chips (A2, A11, A12, A21, A22) nach einem der Ansprüche 1 bis 10 durch einen ersten Farbstoff repräsentiert,
o sowie ferner zum Entgegennehmen einer zweiten Bildinformation (BI2, BI12), welche eine potentielle Färbung der Antigen-Spots (AS) des Antigen-Chips (A2, A11, A12, A21, A22) nach einem der Ansprüche 1 bis 10 durch einen zweiten Farbstoff nach Inkubieren der Spots des Antigen-Chips mit der biologische Probe und mit einem Konjugat, welches einen mit dem zweiten Farbstoff markierten sekundären Antikörper aufweist, repräsentiert,
sowie ferner wenigstens eine Recheneinheit (R2), welche ausgebildet ist,
o auf Basis der wenigstens einen ersten Bildinformation (BI1, BI11) das Referenzmuster (RM) sowie eine zugehörige Referenz-Position zu detektieren,
o auf Basis der wenigstens einen ersten Bildinformation (BI2, BI12) die jeweiligen Antigen-Spot-Muster (AM, AM1, AM2) sowie jeweilige zugehörige weitere Positionen zu detektieren,
∘ auf Basis der detektierten Referenz-Position und der detektierten weiteren Positionen eine Zuordnungsinformation (ZI) zu erstellen, welche eine Zuordnung der jeweiligen Antigen-Spot-Muster zu jeweiligen Antigen-Typen indiziert,
∘ sowie auf Basis der zweiten Bildinformation (BI2, BI12) jeweilige Bindungsmaße zu bestimmen, welche jeweilige Bindungen von Antikörpern der biologischen Probe an jeweilige Antigen-Typen indizieren.

23. Computerprogrammprodukt,
umfassend Befehle, die bei der Ausführung des Programms durch einen Computer diesen veranlassen, ein Verfahren nach einem der Ansprüche 12 bis 15 auszuführen.

24. Datenträgersignal, welches das Computerprogrammprodukt nach Anspruch 23 überträgt.

25. Objektträger (OT),
aufweisend eine Vielzahl von Antigen-Chips (A) nach einem der Ansprüche 1 bis 10.

26. Kit zur Verwendung in einem Verfahren zum Detektieren von Antikörpern in einer Probe, aufweisend:
wenigstens einen Antigen-Chip (A2, A11, A12, A21, A22) nach einem der Ansprüche 1 bis 10,
sowie ferner ein Konjugat, welches einen mit einem zweiten Farbstoff markierten sekundären Antikörper aufweist.

27. Kit nach Anspruch 26,
wobei der zweite Farbstoff ein Fluoreszenzfarbstoff, ein chromogenes Substrat, ein Enzym oder ein Substrat für eine Chemilumenszenzreaktion ist.

## Geänderte Patentansprüche

### Geänderte Patentansprüche gemäss Regel 137(2) EPÜ.

1. Antigen-Chip (A2, A11, A12, A21, A22),
aufweisend eine ebene Substratoberfläche (SO) mit darauf aufgebrachten, zueinander beabstandeten Antigen-Spots (AS), welche einen gleichen, gemeinsamen Farbstoff aufweisen,
wobei die Antigen-Spots (AS) jeweilige Antigen-Spot-Mengen (AG) ausbilden, welche korrespondierende jeweilige, regelmäßige Antigen-Spot-Muster (AM, AM1, AM2) ausbilden,
wobei Antigen-Spots (AS) einer gleichen Antigen-Spot-Menge einen gleichen, gemeinsamen Antigentyp aufweisen und wobei zwei oder mehr der Antigen-Spot-Mengen (A1, A2) unterschiedliche Antigentypen aufweisen,
**dadurch gekennzeichnet, dass** auf der Substratoberfläche (SO) ferner Referenz-Spots (RS) aufgebracht sind, welche eine Referenz-Spot-Menge ausbilden, welche ein regelmäßiges Referenz-Muster (RM) ausbildet, wobei die Referenz-Spots (RS) ebenfalls den gleichen, gemeinsamen Farbstoff aufweisen,
und dass sich das Referenz-Muster (RM) hinsichtlich seiner Regelmäßigkeit von den Antigen-Spot-Mustern (AM, AM1, AM2) durch eines oder mehrere der folgenden Kriterien unterscheidet:
∘ innerhalb des Referenz-Musters (RM) und innerhalb der Antigen-Spot-Muster (AM, AM1, AM2) folgen die jeweils zugehörigen Spots in jeweiligen regelmäßigen Abständen (SD1, SD2) aufeinander, wobei der regelmäßige Abstand (SD1) der Referenz-Spots (RS) des Referenz-Musters (RM) sich von den regelmäßigen Abständen (SD2) der Antigen-Spots der Antigen-Spot-Muster (AM, AM1, AM2) unterscheidet;
∘ das Referenz-Muster (RM) weist zu einem unmittelbar benachbarten Muster einen solchen Nachbar-Abstand auf, welcher sich von den anderen Nachbar-Abständen der Antigen-Spot-Muster unterscheidet;
∘ innerhalb des Referenz-Musters (RM) und innerhalb der Antigen-Spot-Muster (AM, AM1, AM2) weisen die jeweils zugehörigen Spots eine jeweilige, gleiche Größe auf, so dass die Größe der Referenz-Spots des Referenz-Musters (RM) sich von den Größen der Antigen-Spots der Antigen-Spot-Muster (AM, AM1, AM2) unterscheidet.

2. Antigen-Chip (A2, A11, A12, A21, A22) nach Anspruch 1,
**dadurch gekennzeichnet, dass** das Referenzmuster (RM) und die Antigenmuster (AM, AM1, AM2) entlang einer Anordnungsrichtung (ANR) der Muster angeordnet sind,
und dass das Referenzmuster (RM) und die Antigenmuster (AM, AM1, AM2) jeweilige Ausbreitungsrichtungen (ABR) aufweisen, welche im Wesentlichen senkrecht zu der Anordnungsrichtung (ANR) der Muster (RM, AM, AM1, AM2) verläuft.

3. Antigen-Chip (A2, A11, A12, A21, A22) nach Anspruch 1,
**dadurch gekennzeichnet, dass** die Referenz-Spots der Referenzspot-Menge ein Referenz-Linienmuster (RM) ausbilden und dass ferner die jeweiligen Antigen-Spots der jeweiligen Antigen-Spot-Mengen jeweilige Antigen-Linienmuster (AM, AM1, AM2) ausbilden,
wobei die Linienmuster im Wesentlichen parallel zueinander verlaufen.

4. Antigen-Chip (A2, A11, A12, A21, A22) nach Anspruch 1,
**dadurch gekennzeichnet, dass** der gleiche, gemeinsame Farbstoff ein Fluoreszenzfarbstoff ist.

5. Antigen-Chip (A2, A11, A12, A21, A22) nach Anspruch 1,
**dadurch gekennzeichnet, dass** der zweite Farbstoff ein Fluoreszenzfarbstoff ist.

6. Antigen-Chip (A2, A11, A12, A21, A22) nach einem der Ansprüche 1 bis 5,
**dadurch gekennzeichnet, dass** die Referenz-Spots (RS) der Referenz-Spot-Menge den Farbstoff in einer Farbstoffkonzentration aufweisen, welche größer ist als die Farbstoffkonzentrationen, in welchen die Antigen-Spots der Antigen-Spot-Mengen (AM, AM1, AM2) den Farbstoff aufweisen.

7. Antigen-Chip (A2, A11, A12) nach einem der Ansprüche 1 bis 4,
wobei die Referenz-Spots (RS) erste Referenz-Spots sind, welche eine erste Referenz-Spot-Menge ausbilden, welche wiederum ein erstes regelmäßiges Referenz-Muster (RM) ausbildet,
**gekennzeichnet durch** zweite Referenz-Spots, welche eine zweite Referenz-Spot-Menge ausbilden, welche wiederum ein zweites regelmäßiges Referenz-Muster (RM2) ausbildet, wobei die zweiten Referenz-Spots ebenfalls den gleichen, gemeinsamen Farbstoff aufweisen,
ferner **dadurch gekennzeichnet, dass** sich das zweite Referenz-Muster (RM2) von den Antigen-Spot-Mustern (AM, AM1, AM2) der Antigen-Spot-Mengen und zusätzlich auch von dem ersten Referenz-Muster (RM) der ersten Referenz-Spots hinsichtlich seiner Regelmäßigkeit unterscheidet.

8. Antigen-Chip nach einem der Ansprüche 1 bis 5,
wobei die Referenz-Spots (RS) ferner ein Antigen aufweisen.

9. Antigen-Chip nach einem der Ansprüche 1 bis 5,
wobei die Referenz-Spots (RS) ferner einen Antikörper, insbesondere IgG, aufweisen.

10. Verfahren zum automatisierten Detektieren von Antikörpern in einer flüssigen biologischen Probe mittels Verwendung eines Antigen-Chips (A2, A11, A12, A21, A22), aufweisend die Schritte
Bereitstellen eines Antigen-Chips (A2, A11, A12, A21, A22) nach einem der Ansprüche 1 bis 9, (S1)
Inkubieren der Spots des Antigen-Chips (A2, A11, A12, A21, A22) mit der biologischen Probe, (S2)
Inkubieren der Spots mit einem Konjugat, welches einen mit einem zweiten Farbstoff markierten sekundären Antikörper aufweist, (S3) Erfassen oder Bereitstellen wenigstens einer ersten Bildinformation (BI1, BI11),
welche eine Färbung der Referenz-Spots und der Antigen-Spots durch den ersten Farbstoff repräsentiert, (S4)
Detektieren des Referenzmusters (RM) sowie einer zugehörigen Referenz-Position auf Basis der wenigstens einen ersten Bildinformation (BI1, BI11), (S5) Detektieren der jeweiligen Antigen-Spot-Muster (AM, AM1, AM2) sowie jeweiliger zugehöriger weiterer Positionen auf Basis der wenigstens einen ersten Bildinformation (BI1, BI11), (S6)
auf Basis der detektierten Referenz-Position und der detektierten weiteren Positionen, Erstellen einer Zuordnungs-Information (ZI), welche eine Zuordnung der jeweiligen Antigen-Spot-Muster (AM, AM1, AM2) zu jeweiligen Antigen-Typen indiziert, (S7)
Erfassen oder Bereitstellen einer zweiten Bildinformation (BI2, BI12), welche eine potentielle Färbung der Antigen-Spots durch den zweiten Farbstoff repräsentiert, (S8)
auf Basis der zweiten Bildinformation (BI2, BI12) und der Zuordnungs-Information (ZI), Bestimmen jeweiliger Bindungsmaße, welche jeweilige Bindungen von Antikörpern der biologischen Probe an jeweilige Antigen-Typen indizieren. (S9)

11. Verfahren für eine automatisierte Bildverarbeitung zum Detektieren von Antikörpern in einer flüssigen biologischen Probe, aufweisend
Bereitstellen oder Erfassen einer ersten Bildinformation (BI1, BI11), welche eine Färbung von Referenz-Spots und von Antigen-Spots eines Antigen-Chips (A2, A11, A12, A21, A22) nach einem der Ansprüche 1 bis 9 durch einen ersten Farbstoff repräsentiert, (S4)
Detektieren des Referenzmusters (RM) sowie einer zugehörigen Referenz-Position, (S5)
Detektieren der jeweiligen Antigen-Spot-Muster (AM, AM1, AM2) sowie jeweiliger zugehöriger weiterer Positionen auf Basis der wenigstens einen ersten Bildinformation (BI1, BI11), (S6)
auf Basis der detektierten Referenz-Position und der detektierten weiteren Positionen, Erstellen einer Zuordnungsinformation (ZI), welche eine Zuordnung der jeweiligen Antigen-Spot-Muster zu jeweiligen Antigen-Typen indiziert, (S7) Bereitstellen oder Erfassen einer zweiten Bildinformation (BI2, BI12), welche eine potentielle Färbung der Antigen-Spots des Antigen-Chips (A2, A11, A12, A21, A22) durch einen zweiten Farbstoff nach Inkubieren der Spots des Antigen-Chips mit der biologische Probe und mit einem Konjugat, welches einen mit dem zweiten Farbstoff markierten sekundären Antikörper aufweist, repräsentiert, (S8) auf Basis der zweiten Bildinformation (BI2, BI12), Bestimmen jeweiliger Bindungsmaße, welche jeweilige Bindungen von Antikörpern der biologischen Probe an jeweilige Antigen-Typen indizieren. (S9)

12. Verfahren nach Anspruch 10 oder 11,
wobei der Antigen-Chip ein Antigen-Chip nach Anspruch 7 ist,
wobei die Referenz-Position eine erste Referenz-Position ist,
ferner aufweisend:
Detektieren des zweiten Referenzmusters (RM2) sowie einer zweiten zugehörigen Referenz-Position auf Basis der ersten Bildinformation (BI1, BI11), Erstellen der Zuordnungsinformation (ZI), welche die Zuordnung der jeweiligen Antigen-Spot-Muster zu den jeweiligen Antigen-Typen indiziert, auf Basis der detektierten ersten Referenz-Position, der detektierten zweiten Referenz-Position und der detektierten weiteren Positionen.

13. Verfahren nach Anspruch 10 oder 11,
wobei die Zuordnung der jeweiligen detektierten Antigen-Spot-Muster zu jeweiligen Antigen-Typen erfolgt durch
Bestimmen einer räumlichen Abfolge der Referenz-Position und der weiteren Positionen,
Bereitstellen eines Abfolge-Datensatzes (ADS), welcher eine Abfolge von Antigen-Typen indiziert,
Erstellen der Zuordnungsinformation (ZI), welche die Zuordnung der jeweiligen Antigen-Spot-Muster zu den jeweiligen Antigen-Typen indiziert, auf Basis der detektierten Referenz-Position, der detektierten weiteren Positionen und des Abfolge-Datensatzes (ADS).

14. Verfahren nach Anspruch 11,
wobei die erste Bildinformation (BI1, BI11) über wenigstens eine Datenschnittstelle (DS) entgegengenommen wird und mittels wenigstens einer Speichereinheit (MEM) bereitgestellt wird
und wobei die zweite Bildinformation (BI2, BI12) über die Datenschnittstelle (DS) entgegengenommen wird und mittels der Speichereinheit (MEM) bereitgestellt wird.

15. Verfahren zur Herstellung eines Antigen-Chips (A2, A11, A12, A21, A22) zur Immunodiagnostik, aufweisend
∘ Bereitstellen eines Substrats mit einer ebenen Substratoberfläche (SO),
∘ Aufbringen von Referenz-Spots (RS) auf die ebene Substratoberfläche (SO), sowie
∘ Aufbringen von Antigen-Spots (AS) auf die ebene Substratoberfläche (SO),
wobei das Aufbringen der Referenz-Spots und der Antigen-Spots (AS) derart durchgeführt wird, dass die Referenz-Spots (RS) und die Antigen-Spots (AS) des Antigen-Chips (A2, A11, A12, A21, A22) gemäß einem Antigen-Chip nach einem der Ansprüche 1 bis 9 ausgeführt sind.

16. Verfahren nach Anspruch 15,
ferner aufweisend : Fragmentieren des Substrats (SU), um mehrere Antigen-Chips (A11, A12) zu erhalten.

17. Verfahren nach Anspruch 15 oder 16,
wobei die Substratoberfläche (SO) eine Oberfläche eines Glas-Substrates oder eines mit einer Membran und/oder Folie beschichteten Glas-Substrates ist.

18. Vorrichtung (V1) für eine automatisierte Bildverarbeitung zum Detektieren von Antikörpern in einer flüssigen biologischen Probe, aufweisend
wenigstens eine Bilderfassungseinheit (K1, K2)
∘ zum Erfassen einer ersten Bildinformation (BI1, BI11), welche eine Färbung von Referenz-Spots (RS) und von Antigen-Spots (AS) eines Antigen-Chips (A2, A11, A12, A21, A22) nach einem der Ansprüche 1 bis 9 durch einen ersten Farbstoff repräsentiert,
∘ sowie ferner zum Erfassen einer zweiten Bildinformation (BI2, BI12), welche eine potentielle Färbung der Antigen-Spots (AS) des Antigen-Chips (A2, A11, A12, A21, A22) nach einem der Ansprüche 1 bis 9 durch einen zweiten Farbstoff nach Inkubieren der Spots des Antigen-Chips mit der biologische Probe und mit einem Konjugat, welches einen mit dem zweiten Farbstoff markierten sekundären Antikörper aufweist, repräsentiert,
sowie ferner wenigstens eine Recheneinheit (R), welche ausgebildet ist,
∘ auf Basis der wenigstens einen ersten Bildinformation (BI1, BI11) das Referenzmuster (RM) sowie eine zugehörige Referenz-Position zu detektieren,
∘ auf Basis der wenigstens einen ersten Bildinformation (BI1, BI11) die jeweiligen Antigen-Spot-Muster (AM) sowie jeweilige zugehörige weitere Positionen zu detektieren,
∘ auf Basis der detektierten Referenz-Position und der detektierten weiteren Positionen eine Zuordnungsinformation (ZI) zu erstellen, welche eine Zuordnung der jeweiligen Antigen-Spot-Muster zu jeweiligen Antigen-Typen indiziert,
∘ sowie auf Basis der zweiten Bildinformation (BI2, BI12) jeweilige Bindungsmaße zu bestimmen, welche jeweilige Bindungen von Antikörpern der biologischen Probe an jeweilige Antigen-Typen indizieren.

19. Vorrichtung nach Anspruch 18,
ferner aufweisend
- wenigstens eine Beleuchtungseinheit (FL) zum Aussenden von Anregungslicht (AL) zum Anregen einer Emission von erster Fluoreszenzstrahlung eines ersten Wellenlängenbereiches durch den ersten Farbstoff.

20. Vorrichtung nach Anspruch 18,
ferner aufweisend
- wenigstens eine Beleuchtungseinheit (FL) zum Aussenden von Anregungslicht (AL) zum Anregen einer Emission von zweiter Fluoreszenzstrahlung eines zweiten Wellenlängenbereiches durch den zweiten Farbstoff.

21. Datennetzwerkvorrichtung (V2) für eine automatisierte Bildverarbeitung zum Detektieren von Antikörpern in einer flüssigen biologischen Probe, aufweisend
wenigstens eine Datenschnittstelle (DS)
∘ zum Entgegennehmen einer ersten Bildinformation (BI1, BI11), welche eine Färbung von Referenz-Spots (RS) und von Antigen-Spots (AS) eines Antigen-Chips (A2, A11, A12, A21, A22) nach einem der Ansprüche 1 bis 9 durch einen ersten Farbstoff repräsentiert,
∘ sowie ferner zum Entgegennehmen einer zweiten Bildinformation (BI2, BI12), welche eine potentielle Färbung der Antigen-Spots (AS) des Antigen-Chips (A2, A11, A12, A21, A22) nach einem der Ansprüche 1 bis 9 durch einen zweiten Farbstoff nach Inkubieren der Spots des Antigen-Chips mit der biologische Probe und mit einem Konjugat, welches einen mit dem zweiten Farbstoff markierten sekundären Antikörper aufweist, repräsentiert,
sowie ferner wenigstens eine Recheneinheit (R2), welche ausgebildet ist,
∘ auf Basis der wenigstens einen ersten Bildinformation (BI1, BI11) das Referenzmuster (RM) sowie eine zugehörige Referenz-Position zu detektieren,
∘ auf Basis der wenigstens einen ersten Bildinformation (BI2, BI12) die jeweiligen Antigen-Spot-Muster (AM, AM1, AM2) sowie jeweilige zugehörige weitere Positionen zu detektieren,
∘ auf Basis der detektierten Referenz-Position und der detektierten weiteren Positionen eine Zuordnungsinformation (ZI) zu erstellen, welche eine Zuordnung der jeweiligen Antigen-Spot-Muster zu jeweiligen Antigen-Typen indiziert,
∘ sowie auf Basis der zweiten Bildinformation (BI2, BI12) jeweilige Bindungsmaße zu bestimmen, welche jeweilige Bindungen von Antikörpern der biologischen Probe an jeweilige Antigen-Typen indizieren.

22. Computerprogrammprodukt,
umfassend Befehle, die bei der Ausführung des Programms durch einen Computer diesen veranlassen, ein Verfahren nach einem der Ansprüche 11 bis 14 auszuführen.

23. Datenträgersignal, welches das Computerprogrammprodukt nach Anspruch 22 überträgt.

24. Objektträger (OT),
aufweisend eine Vielzahl von Antigen-Chips (A) nach einem der Ansprüche 1 bis 9.

25. Kit zur Verwendung in einem Verfahren zum Detektieren von Antikörpern in einer Probe, aufweisend:
wenigstens einen Antigen-Chip (A2, A11, A12, A21, A22) nach einem der Ansprüche 1 bis 9,
sowie ferner ein Konjugat, welches einen mit einem zweiten Farbstoff markierten sekundären Antikörper aufweist.

26. Kit nach Anspruch 25,
wobei der zweite Farbstoff ein Fluoreszenzfarbstoff, ein chromogenes Substrat, ein Enzym oder ein Substrat für eine Chemilumenszenzreaktion ist.
